# EUROPEAN PATENT APPLICATION

(11) **EP 2 810 655 A1**
(43) Date of publication of application: **10.12.2014**
(21) Application number: 13740969.4
(22) Date of filing: 28.01.2013
(51) Int. Cl.: A61K 45/00, A61K 31/7088, A61K 31/711, A61K 48/00, A61P 35/00, C12N 15/113, C12Q 1/68, G01N 33/15, G01N 33/50, G01N 33/574

(54) **NOVEL ANTITUMOR AGENT AND METHOD FOR SCREENING SAME**

(30) Priority: 27.01.2012 JP 2012015982
(71) Applicant: Osaka University, Suita-shi, Osaka 565-0871 (JP)
(72) Inventor: ISHII, Masaru, Suita-shi Osaka 565-0871 (JP); KAGAWA, Yoshinori, Suita-shi Osaka 565-0871 (JP); MORI, Masaki, Suita-shi Osaka 565-0871 (JP); ISHII, Hideshi, Suita-shi Osaka 565-0871 (JP)
(74) Representative: De Clercq, Ann G. Y.
(86) International application number: PCT/JP2013/051733
(87) International publication number: WO 2013/111897

(57) **Abstract**

The present invention provides a novel antitumor agent that acts through a novel mechanism. The novel antitumor agent contains as an active ingredient a substance capable of inhibiting a cell cycle-dependent Rho GTPase activating protein (RhoGAP). The RhoGAP is cell cycle-dependent and plays an important role in a process through which cancer cells acquire invasive capacity and/or metastatic capacity. The invasion and/or metastasis of cancer cells can be controlled by targeting the RhoGAP. Examples of the substance capable of inhibiting a cell cycle-dependent RhoGAP include an antisense oligonucleotide against a gene encoding the RhoGAP and an oligonucleotide that induces RNA interference of the gene. As the oligonucleotide, one containing an artificial nucleic acid such as BNA is preferred because of its excellent stability. The present invention also provides a screening method including selecting an antitumor agent capable of suppressing cancer cell invasion and/or metastasis through a novel mechanism. The screening method is a screening method for a novel antitumor agent, including selecting a substance capable of inhibiting a cell cycle-dependent Rho GTPase activating protein.

## Description

### Technical Field

The present invention relates to a novel antitumor agent that targets a cell cycle-dependent protein and acts through a novel mechanism. The present invention also relates to a screening method including selecting an antitumor agent capable of suppressing cancer cell invasion and/or metastasis through a novel mechanism.

The present application claims priority from Japanese Patent Application No. 2012-015982, which is incorporated herein by reference.

### Background Art

When cells proliferate, the cells undergo a regular process called a cell cycle. That is, the cells proliferate by regularly repeating the cell cycle in the order of G1 phase (gap 1)→S phase (DNA synthesis)→G2 phase (gap 2)→M phase (mitosis)→G1 phase. One important point for progression of the cell cycle exists at the boundary between the G1 phase and the S phase. In mammalian cultured cells, this point is called a restriction point (R point). Once having passed the R point, the cell cycle is directed to progress. Then, the cells immediately enter the S phase, then enter the G2 phase and the Mphase, and return to the G1 phase. Under an environment in which the cells do not proliferate, the cells remain in the G1 phase without entering the S phase or exit the cell cycle to enter a special state called a resting phase (resting state; G0 phase), falling in a quiescent state. Depending on the environment in which the cells are placed, the cells may receive a signal for progression to differentiation, senescence, apoptosis, meiosis, or the like. It is currently considered that a branching point leading to these states also exists before the R point of the G1 phase.

Cancer cells are cells with abnormal cell cycle control that continue proliferating while ignoring a signal for division arrest derived from surrounding cells. The cancer cells proliferate unlimitedly and eventually cause dysfunction of organs throughout the body by invasion/metastasis, leading to the death of a patient. Hitherto, therapeutic drugs for a malignant tumor, such as a conventionally used anti-malignant tumor agent and a cancer cell-specific molecular-targeted drug for suppressing a proliferation signal, have each been one for impeding highly proliferative cancer cells. In addition, recently, there has also been available a therapy involving suppressing angiogenesis around a malignant tumor tissue to cut off a supply route to the malignant tumor tissue engaged in active metabolism. Specific examples thereof include: trastuzumab, which targets HER2 expressed in breast cancer; gefitinib, which inhibits a kinase activity of an epidermal growth factor receptor (EGFR); imatinib mesylate, which inhibits a tyrosine kinase activity of Bcr-Abl chimeric gene derived from a chromosomal translocation in chronic myelogenous leukemia (CML); tuximab, which recognizes CD20 antigen specific for B-cell lymphoma; rituximab, which contains a monoclonal antibody against cell surface antigen CD33 expressed in acute myelogenous leukemia cells (AML); erlotinib, which inhibits tyrosine kinase enzyme of EGFR; and bevacizumab, which is formed of a monoclonal antibody against a vascular endothelial growth factor (VEGF). There has also been used bortezomib, which inhibits a protein required for cell cycle progression to induce apoptosis. In addition, there has also been developed a therapy involving enhancing antitumor immunity of a host, such as a vaccine therapy.

Meanwhile, the most important process through which a malignant tumor leads to a fatal condition is invasion/metastasis. In particular, even when cells that have become cancerous are locally generated, there are various structural restrictions on the proliferation of the cells *in vivo,* and hence without sufficient invasive capacity, the cancer cells are locally confined and unable toproliferate/progress. The cancer cells separate from the primary tumor, and invade vessels by destroying the surrounding stroma and basement membrane through production of a protease. The cancer cells then migrate through the vessels to adhere to vascular endothelial cells of a target organ, and invade a tissue through blood vessels on the basis of a similar mechanism. It is considered that the cancer cells then proliferate at the site to form a metastasis focus. In recent years, it has been considered that a cell adhesion molecule and a special protease play large roles in the processes of the separation from the primary tumor and the adhesion to and invasion into the target tissue.

However, drugs for suppressing cancer cell invasion and/or metastasis cannot be said to be sufficient, and there is a demand for further development.

### Citation List

### Non Patent Literature

[NPL1] NK4 (HGF-antagonist/angiogenesisinhibitor) in cancer biology and therapeutics. Matsumoto K, Nakamura T. (2003) Cancer Science 94: 321-327.

### Summary of Invention

### Technical Problem

An object of the present invention is to provide a novel antitumor agent that targets a cell cycle-dependent protein and acts through a novel mechanism. Another object of the present invention is to provide a screening method including selecting an antitumor agent capable of suppressing cancer cell invasion and/or metastasis through a novel mechanism.

### Solution to Problem

The inventors of the present invention have made extensive studies in order to achieve the objects. As a result, the inventors have found for the first time that a certain Rho GTPase activating protein (RhoGAP, herein after sometimes referred to simply as "RhoGAP") is regulated in a cell cycle-dependent manner and plays an important role in a process through which cancer cells acquire invasive capacity (cell mobility). The inventors have confirmed that the mobility and/or metastasis of cancer cells can be controlled by targeting the RhoGAP. Thus, the present invention has been completed.

That is, the present invention includes the following.
1. A novel antitumor agent, including as an active ingredient a substance capable of inhibiting a cell cycle-dependent Rho GTPase activating protein (RhoGAP).
2. A novel antitumor agent according to the above-mentioned item 1, in which the substance capable of inhibiting a cell cycle-dependent RhoGAP includes a substance capable of inhibiting ARHGAP11A.
3. A novel antitumor agent according to the above-mentioned item 2, in which the substance capable of inhibiting ARHGAP11A includes any one selected from: an antisense oligonucleotide against a gene encoding the ARHGAP11A; an oligonucleotide that induces RNA interference of the gene encoding the ARHGAP11A; and a low-molecular-weight compound and a natural polymer each capable of binding to a transcription product or translation product of the gene encoding the ARHGAP11A.
4. A novel antitumor agent according to the above-mentioned item 3, in which the substance capable of inhibiting ARHGAP11A includes the antisense oligonucleotide against the gene encoding the ARHGAP11A, the oligonucleotide including from 14 bases to 200 bases, the base sequence including at least one or more artificial nucleic acids.
5. A novel antitumor agent according to the above-mentioned item 4, in which the antisense oligonucleotide includes an oligonucleotide including a base sequence set forth in any one of the following items 1) to 13):
   1) ARHGAP-625-BNA-16: 5(L)T(L)5(L)aaatttgaa5(L)T(L)5(L)c (SEQ ID NO: 10);
   2) ARHGAP-969-BNA-16: T(L)5(L)5(L)gaaaaagcc5(L)T(L)T(L)c (SEQ ID NO: 13);
   3) ARHGAP-1344-BNA-16: T(L)5(L)T(L)tttcatgtc5(L)T(L)T(L)c (SEQ ID NO: 16);
   4) ARHGAP-1447-BNA-16: T(L)5(L)5(L)aggataaaaT(L)5(L)T(L)g (SEQ ID NO: 17);
   5) ARHGAP-1748-BNA-16: 5(L)T(L)T(L)gatggactt5(L)5(L)T(L)t (SEQ ID NO: 19);
   6) ARHGAP-1931-BNA-16: T(L)T(L)T(L)gcctgcaatT(L)5(L)T(L)t (SEQ ID NO: 21);
   7) ARHGAP-2032-BNA-16: 5(L)5(L)T(L)agattgaatT(L)T(L)5(L)a (SEQ ID NO: 22);
   8) ARHGAPv1-3484-BNA-16: T(L)T(L)5(L)gagggtaacT(L)5(L)5(L)a (SEQ ID NO: 30);
   9) ARHGAPv2-2215-BNA-16: 5(L)T(L)5(L)taacagtagT(L)A(L)T(L)g (SEQ ID NO: 34);
   10) ARHGAPv2-2285-BNA-16: T(L)5(L)T(L)agaacagtaA(L)A(L)T(L)t (SEQ ID NO: 35);
   11) ARHGAPv2-2306-BNA-16: T(L)T(L)5(L)aaacatgaa5(L)T(L)T(L)t (SEQ ID NO: 36);
   12) ARHGAPv2-2355-BNA-16: T(L)5(L)5(L)caattgttgA(L)T(L)A(L)g (SEQ ID NO: 37); and
   13) ARHGAPv2-2404-BNA-16: T(L)T(L)T(L)taacataagA(L)A(L)T(L)g (SEQ ID NO: 38),
      where N (L) represents artificial nucleic acid BNA, 5 (L) represents L-mC (methylated artificial nucleic acid BNA), T(L) represents artificial nucleic acid thymidine, and A(L) represents artificial nucleic acid adenine.
6. A novel antitumor agent according to the above-mentioned item 3, in which the substance capable of inhibiting ARHGAP11A includes the oligonucleotide that induces RNA interference, the oligonucleotide including a base sequence set forth in the following item 14) or 15):
   14) ARHGAP11A #1 (TRCN0000047281): CCGGCGGTATCAGTTCACATCGATACTCGAGTATCGATGTGAACTGATACCGTTTTTG (SEQ ID NO: 1); or
   15) ARHGAP11A #2 (TRCN0000047282): CCGGCCTTCTATTACACCTCAAGAACTCGAGTTCTTGAGGTGTAATAGAAGGTTTTTG (SEQ ID NO: 2).
7. A novel antitumor agent according to any one of the above-mentioned items 1 to 6, in which the antitumor agent has a metastasis inhibitory action on a malignant tumor and/or an invasion inhibitory action on a malignant tumor.
8. A screening method for a novel antitumor agent, including selecting a substance capable of inhibiting a cell cycle-dependent RhoGAP.
9. A screening method according to the above-mentioned item 8, in which the cell cycle-dependent RhoGAP includes ARHGAP11A.
10. A screening method according to the above-mentioned item 9, including at least the following steps A) to D):
   A) a step of culturing a candidate substance together with a cancer cell line, followed by measurement of an expression level of a gene encoding the ARHGAP11A in the cell;
   B) a step of measuring an expression level of the gene encoding the ARHGAP11A measured by the same technique as that of the step A) in a control system;
   C) a step of comparing the expression levels of the gene encoding the ARHGAP11A measured in the steps A) and B) to each other; and
   D) a step of selecting a candidate substance in a case where the expression level of the gene measured in the step A) is lower than the expression level of the gene measured in the step B).
11. A method of testing for a malignant tumor, including quantifying a cell cycle-dependent RhoGAP in a biological specimen.
12. A method of testing for a malignant tumor according to the above-mentioned item 11, in which the cell cycle-dependent RhoGAP includes ARHGAP11A.
13. A method of testing for a malignant tumor according to the above-mentioned item 11 or 12, in which the malignant tumor includes one or more kinds selected from colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, and skin cancer.
14. A method of testing for a malignant tumor according to any one of the above-mentioned items 11 to 13, in which the testing for the malignant tumor includes a test for predicting a stage of progression and/or prognosis of cancer.
15. A metastasis inhibitor and/or invasion inhibitor for a malignant tumor, including a substance capable of inhibiting a cell cycle-dependent RhoGAP.
16. A metastasis inhibitor and/or invasion inhibitor for a malignant tumor according to the above-mentioned item 15, in which the substance capable of inhibiting a cell cycle-dependent RhoGAP includes a substance capable of inhibiting ARHGAP11A.
17. A metastasis inhibitor and/or invasion inhibitor for a malignant tumor according to the above-mentioned item 16, in which the substance capable of inhibiting ARHGAP11A includes any one selected from: an antisense oligonucleotide against a gene encoding the ARHGAP11A; an oligonucleotide that induces RNA interference of the gene encoding the ARHGAP11A; and a low-molecular-weight compound and a natural polymer each capable of binding to a transcription product or translation product of the gene encoding the ARHGAP11A.
18. A metastasis inhibitor and/or invasion inhibitor for a malignant tumor according to the above-mentioned item 17, in which the substance capable of inhibiting ARHGAP11A includes the antisense oligonucleotide against the gene encoding the ARHGAP11A, the oligonucleotide including from 14 bases to 200 bases, the base sequence including at least one or more artificial nucleic acids.
19. A metastasis inhibitor and/or invasion inhibitor for a malignant tumor according to the above-mentioned item 18, in which the antisense oligonucleotide includes an oligonucleotide including a base sequence set forth in any one of the following items 1) to 13):
   1) ARHGAP-625-BNA-16: 5(L)T(L)5(L)aaatttgaa5(L)T(L)5(L)c (SEQ ID NO: 10);
   2) ARHGAP-969-BNA-16: T(L)5(L)5(L)gaaaaagcc5(L)T(L)T(L)c (SEQ ID NO: 13);
   3) ARHGAP-1344-BNA-16: T(L)5(L)T(L)tttcatgtc5(L)T(L)T(L)c (SEQ ID NO: 16);
   4) ARHGAP-1447-BNA-16: T(L)5(L)5(L)aggataaaaT(L)5(L)T(L)g (SEQ ID NO: 17);
   5) ARHGAP-1748-BNA-16: 5(L)T(L)T(L)gatggactt5(L)5(L)T(L)t (SEQ ID NO: 19);
   6) ARHGAP-1931-BNA-16: T(L)T(L)T(L)gcctgcaatT(L)5(L)T(L)t (SEQ ID NO: 21);
   7) ARHGAP-2032-BNA-16: 5(L)5(L)T(L)agattgaatT(L)T(L)5(L)a (SEQ ID NO: 22);
   8) ARHGAPv1-3484-BNA-16: T(L)T(L)5(L)gagggtaacT(L)5(L)5(L)a (SEQ ID NO: 30);
   9) ARHGAPv2-2215-BNA-16: 5(L)T(L)5(L)taacagtagT(L)A(L)T(L)g (SEQ ID NO: 34);
   10) ARHGAPv2-2285-BNA-16: T(L)5(L)T(L)agaacagtaA(L)A(L)T(L)t (SEQ ID NO: 35);
   11) ARHGAPv2-2306-BNA-16: T(L)T(L)5(L)aaacatgaa5(L)T(L)T(L)t (SEQ ID NO: 36);
   12) ARHGAPv2-2355-BNA-16: T(L)5(L)5(L)caattgttgA(L)T(L)A(L)g (SEQ ID NO: 37); and
   13) ARHGAPv2-2404-BNA-16: T(L)T(L)T(L)taacataagA(L)A(L)T(L)g (SEQ ID NO: 38),
      where N (L) represents artificial nucleic acid BNA, 5 (L) represents L-mC (methylated artificial nucleic acid BNA), T(L) represents artificial nucleic acid thymidine, and A(L) represents artificial nucleic acid adenine.
20. Ametastasis inhibitor and/or invasion inhibitor for a malignant tumor according to the above-mentioned item 17, in which the substance capable of inhibiting ARHGAP11A includes the oligonucleotide that induces RNA interference, the oligonucleotide including a base sequence set forth in the following item 14) or 15):
   14) ARHGAP11A #1 (TRCN0000047281): CCGGCGGTATCAGTTCACATCGATACTCGAGTATCGATGTGAACTGATACCGTTTTTG (SEQ ID NO: 1); or
   15) ARHGAP11A #2 (TRCN0000047282): CCGGCCTTCTATTACACCTCAAGAACTCGAGTTCTTGAGGTGTAATAGAAGGTTTTTG (SEQ ID NO: 2).
21. A therapeutic and/or prophylactic method for a malignant tumor, including using the novel antitumor agent according to any one of the above-mentioned items 1 to 7.

### Advantageous Effects of Invention

The novel antitumor agent containing as an active ingredient a substance capable of inhibiting a cell cycle-dependent RhoGAP of the present invention suppresses cancer cell invasion and/or metastasis in, for example, cancer cells derived from cancer capable of expressing ARHGAP11A, such as colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, or skin cancer. Hitherto, there has been almost no effective drug that is an antitumor agent capable of suppressing cancer cell invasion and/or metastasis. The provision of the drug capable of suppressing cancer cell invasion and/or metastasis affecting malignant progression of cancer enables an effective cancer therapy.

Further, as a result of testing the expression of ARHGAP11A in a biological specimen on the basis of, for example, mRNA, significant differences have been found in association with the stage of cancer progression (staging) and relapse free survival. Thus, a test for a malignant tumor can be performed by quantifying the cell cycle-dependent RhoGAP in a biological specimen.

### Brief Description of Drawings

FIG. 1 is a photographic view showing a tissue based on tissue immunity of metastatic/invasive cancer cells in a cancer tissue (Reference Example 1).
FIGS. 2 are photographic views confirming the gene introduction of Fucci for real-time visualization of a cell cycle into human colorectal cancer cells by a fluorescence microscope and FACS (Reference Example 2).
FIG. 3 is a photographic view confirming that human colorectal cancer cells implanted into a NOD/SCID mouse have different cell tracking velocities depending on the cell cycle of the cells. Cells in the S/G2/M phases have a higher cell mobility as compared to cells in the G1 phase (Reference Example 2).
FIGS. 4 are photographic views confirming the expression of ARHGAP11A at the mRNA level and as a protein for cells in the S/G2/M phases (mAG) and cells in the G1 phase (mKO2) (Reference Example 2).
FIGS. 5 illustrate a relationship between the presence of a cell cycle-dependent transcription factor and the expression of ARHGAP11A (Reference Example 3).
FIG. 6 is a graph confirming the expression of ARHGAP11A in various cells (Reference Example 4).
FIGS. 7 are graphs confirming cell proliferation rates and mobilities for mutant lines of human colorectal cancer cells in which ARHGAP11A is inhibited by shRNA (Example 1).
FIG. 8 is a graph showing the results of confirming the size of a tumor in the case of implanting human colorectal cancer cells of a wild-type line into an immunocompromised mouse (NOD/SCID), and then locally administering shRNA against ARHGA11A (Example 2).
FIGS. 9 are graphs confirming the expression of ARHGAP11A at the mRNA level against the stage of colorectal cancer (Example 3).
FIG. 10 is a graph showing the results of confirming the relapse free survivals of patients who have undergone the surgical resection of colorectal cancer in a patient group with lower than average ARHGAP11A expression (n=38) and a patient group with higher than average ARHGAP11A expression (n=26) (Example 4).
FIG. 11 is a graph showing the results of confirming suppressive effects on the expression of ARHGAP11A exhibited by 35 kinds of artificial nucleic acid-containing antisense oligonucleotides (antisense BNAs) in HCT116 (human colorectal cancer cell line) (Example 5).
FIGS. 12 are photographic views showing the results of confirming suppressive effects on the expression of ARHGAP11A exhibited by 5 kinds of artificial nucleic acid-containing antisense oligonucleotides (antisense BNAs) in various cancer cells by western blotting (Example 6).
FIGS. 13(A) are photographic views confirming suppressive effects on the expression of ARHGAP11A in HCT116 having introduced therein antisense BNA (#1748). In addition, FIGS. 13(B) are photographic views confirming the incorporation of antisense BNA (#1748) into HCT116 using fluorescein isothiocyanate (FITC)-labeled antisense BNA (#1748) (Example 7).
FIG. 14 is a graph showing an *in vivo* antitumor effect exhibited by FITC-labeled antisense BNA (#1748) administered into an immunocompromised mouse (NOD/SCID) (Example 8).
FIGS. 15 are photographic views showing the results of confirming the incorporation of an FITC label into various organs on day 14 after the administration of FITC-labeled antisense BNA (#1748) into an immunocompromised mouse (NOD/SCID) (Example 8).
FIG. 16 is a diagram illustrating an administration schedule of drugs, for confirming a tumor metastasis suppressive effect of antisense BNA (#1748) (Example 9).
FIGS. 17 are photographic views showing the results of confirming the size of a lung metastasis tumor by imaging in the case of administering antisense BNA (#1748) into a nude mouse into which human fibrosarcoma cells have been intravenously injected (Example 9).
FIGS. 18 are graphs showing the results of measuring lung weights and body weights in the case of administering antisense BNA (#1748) into nude mice to which human fibrosarcoma cells have been intravenously injected (Example 9).

### Description of Embodiments

The present invention relates to a novel antitumor agent containing as an active ingredient a substance capable of inhibiting a cell cycle-dependent RhoGAP. In the present invention, an example of the cell cycle-dependent RhoGAP is Rho GTPase activating protein 11A (ARHGAP11A).

While specific experimental results are described in detail in Examples and experimental examples to be described later, how the present invention has been made is briefly described in this section. The inventors of the present invention have used an intravital imaging experimental system for a cancer tissue established by themselves to perform real-time analysis of the manner in which cancer cells invade a normal tissue, and have found that cells in the S/G2/M phases have a higher mobility as compared to cells in the G1 phase. The inventors have sorted the S/G2/M cells and the G1 cells out of cancer cells and subjected the cells to microarray analysis, to thereby extract cell cycle-dependent genes, and have found for the first time that among the genes, ARHGAP11A associated with the mobility is expressed at a high level in the cells in the S/G2/Mphases. The inventors have confirmed that cancer cells in which ARHGAP11A is inhibited through the use of small hairpin RNA (shRNA) have a lower mobility than that of cancer cells of a wild-type line and thus are reduced in tumor progression rate. Further, tumor expansion has been able to be significantly suppressed by implanting cancer cells of the wild-type line into immunocompromised mice (NOD/SCID), followed by *in vivo* local administration of small interfering RNA (siRNA) against ARHGA11A.

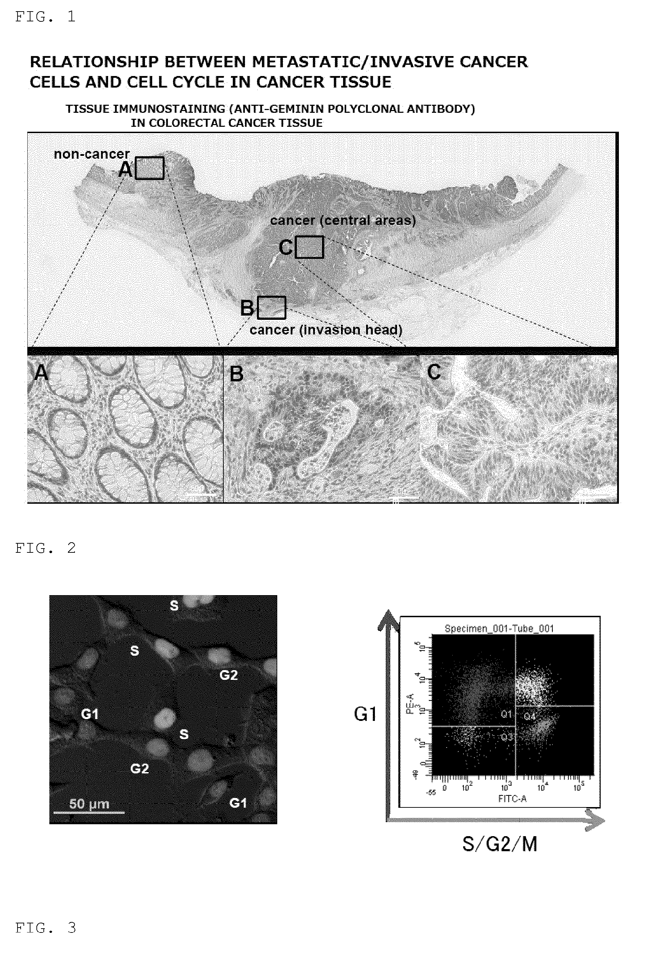

Cancer cells are free of spatial restrictions when proliferating in an incubator, but in order to proliferate *in vivo,* the cancer cells need to invade surrounding normal tissues simultaneously with cell division. The inventors of the present invention have incorporated a fluorescent protein for visualizing the cell cycle of colorectal cancer cells and have performed intravital imaging using a multiphoton excitation microscope. As a result, the inventors have found for the first time that the ability of cancer cells to invade/migrate, i.e., cell mobility is enhanced in the S/G2/M phases depending on the cell cycle (FIGS. 2 and 3). As a result of analyzing molecules expressed in cells in the G1 phase and cells in the S/G2/M phases by a microarray method, it has been confirmed that "ARHGAP11A" as one kind of Rho GTPase activating protein (RhoGAP), which reduces the cell mobility, is expressed in the cells in the S/G2/M phases at a level 10-fold or more higher than in the cells in the G1 phase.

In the present invention, a typical example of ARHGAP11A is a human-derived protein synthesized from mRNA having a base sequence identified by GenBank Accession No. NM_014783.3 (variant 1) or GenBank Accession No. NM_199357.1 (variant 2).In addition, when defined from a different perspective, ARHGAP11A includes a protein having an amino acid sequence translated from the base sequence identified by GenBank Accession No.NM_014783.3 or NM_199357**.**1, or an amino acid sequence having, in the above-mentioned amino acid sequence, deletions, additions, substitutions, or insertions of one or more amino acids, and having cell cycle-dependent Rho GTPase activating property. Further, the ARHGAP11A of the present invention may also encompass ARHGAP11A derived from a non-human mammal.

In the present invention, an ARHGAP11A gene may encompass not only DNA that encodes mRNA having the base sequence identified by GenBank Accession No. NM_014783.3 or NM_199357.1, but also DNA having a similar sequence to these sequences. In this context, the "similar sequence" maybe DNA having a base sequence having deletions, additions, substitutions, or insertions of one or more nucleotides in the base sequence identified by GenBank Accession No. NM_014783.3 or NM_199357.1, and being capable of synthesizing a protein having a cell cycle-dependent RhoGAP activity. Further, the ARHGAP11A gene of the present invention also encompasses: DNA having the base sequence identified above; or DNA having a base sequence having deletions, additions, substitutions, or insertions of one or more nucleotides in the identified base sequence, and being capable of synthesizing a protein having a cell cycle-dependent RhoGAP activity; or DNA having a sequence capable of hybridizing with a partial sequence of the above-mentioned sequence under stringent conditions. The stringent conditions are generally defined by the salt concentration and temperature of a buffer to be used in hybridization or in washing. The salt concentration maybe generally "1×SSC, 0.1% SDS", "0.5×SSC, 0.1% SDS" as amore stringent condition, or "0.1×SSC, 0.1% SDS" as a still more stringent condition. The temperature may be generally 37°C, 42°C as a stringent condition, 55°C as a more stringent condition, or 65°C as a still more stringent condition.

Herein, the substance capable of inhibiting the cell cycle-dependent Rho GTPase activating protein, that is, the cell cycle-dependent RhoGAP refers to a substance that inhibits the function of the RhoGAP and/or inhibits the expression of the RhoGAP. The inhibition of the function of the RhoGAP refers to the inhibition of the function of the RhoGAP as a protein, and the inhibition of the expression of the RhoGAP has the same meaning as the inhibition of the biosynthesis of the RhoGAP. A substance capable of inhibiting the biosynthesis of the RhoGAP is, for example, a substance capable of binding to a RhoGAP gene, or a transcription product or translation product thereof. Specific examples of the transcription product of the RhoGAP gene include RhoGAP mRNA and precursor RNA. An antitumor effect can be expected of the substance capable of inhibiting the biosynthesis of the RhoGAP. A more specific example thereof is any one selected from an antisense oligonucleotide against a gene encoding the RhoGAP, an oligonucleotide that induces RNA interference (RNAi) of the gene (shRNA, siRNA, or microRNA), and a low-molecular-weight compound or natural polymer capable of binding to a transcription product or translation product of the gene.

When the RhoGAP is ARHGAP11A, the substance capable of inhibiting ARHGAP11A refers to a substance capable of inhibiting the function of the ARHGAP11A and/or inhibiting the expression of the ARHGAP11A. The inhibition of the function of the ARHGAP11A refers to the inhibition of the function of the ARHGAP11A as a protein, and the inhibition of the expression of the ARHGAP11A has the same meaning as the inhibition of the biosynthesis of the ARHGAP11A. A substance capable of inhibiting the biosynthesis of the ARHGAP11A is, for example, a substance capable of binding to an ARHGAP11A gene, or a transcription product or translation product thereof. A more specific example thereof is any one selected from an antisense oligonucleotide against a gene encoding the RhoGAP, an oligonucleotide that induces RNA interference (RNAi) of the gene (shRNA, siRNA, or microRNA), and a low-molecular-weight compound or natural polymer capable of binding to a transcription product or translation product of the gene. It should be noted that herein, the "nucleic acid", "nucleotide", and "nucleoside" include not only a deoxyribonucleic acid (DNA) and a ribonucleic acid (RNA), but also artificially synthesized nucleic acids (hereinafter referred to as "artificial nucleic acid") such as a peptide nucleic acid (PNA), a bridged nucleic acid (BNA), and analogs thereof. As the BNA, for example, there may be used a nucleic acid produced by GeneDesign Inc. (bridged nucleic acid).

The sequence of the antisense oligonucleotide may be designed, for example, on the basis of the sequence of the ARHGAP11A gene transcription product so that the antisense oligonucleotide can hybridize with the transcription product. The antisense nucleotide may be constituted of DNA or RNA, and is suitably an oligonucleotide containing an artificial nucleic acid that does not occur naturally. An oligonucleotide having higher stability can be obtained by incorporating the artificial nucleic acid. In addition, the length of the base sequence of the oligonucleotide is not particularly limited and has only to be a length that allows the retention of at least specificity for a gene of interest. The upper limit of the length may be set to a length comparable that of mRNA. The length may be generally from 14 bases to 200 bases, preferably from 15 bases to 50 bases.

Double-stranded RNA (dsRNA) that induces RNA interference (RNAi) is known to have forms such as siRNA, shRNA, and microRNA, and the present invention may encompass all of such forms. The dsRNA is constituted of an antisense strand that can be complementary to a partial region of the transcription product of the ARHGAP11A gene, and a sense strand having a sequence capable of hybridizing with the antisense strand. That is, the double-stranded region of the dsRNA corresponds to a sequence obtained by changing the DNA sequence of a target region on the ARHGAP11A gene to a ribonucleic acid. It should be noted that as long as the expression of ARHGAP11A can be inhibited, the sequence of the antisense strand may contain a sequence not complementary to the target ARHGAP11A gene, and the sequence of the sense strand may contain a sequence that does not hybridize with the antisense strand. The length of the double-stranded region of the dsRNA may be, for example, from 16 to 49 base pairs, suitably from 16 to 30 base pairs, more suitably from 19 to 21 base pairs. In view of the fact that long dsRNA has cytotoxicity, the upper limit of the length may be determined to the extent that the toxicity is not provided. In addition, the lower limit of the length of the dsRNA may be adjusted to the extent that specificity can be retained.

The sequence of, for example, the target region on the ARHGAP11A gene or specific shRNA, siRNA, or microRNA for effectively inhibiting the expression of the cell cycle-dependent RhoGAP such as ARHGAP11A may be designed by utilizing a design tool known per se available from, for example, DHARMACON (http://design.dharmacon.com), Ambion (http://www.ambion.com/techlib/misc/siRNA_finder.html), or TAKARABIOINC. (http://www.takara-bio.co.jp/rnai/intro.htm). In addition, artificially synthesized synthetic oligo dsRNA may be prepared by means of a synthesizer or the like. Alternatively, there may be adopted a construction in which siRNA is expressed in cells from an expression vector carrying DNA that encodes dsRNA. The vector for expressing dsRNA in cells may be arbitrarily selected depending on, for example, the cells into which the vector is to be introduced. For example, examples of the vector in mammalian cells include virus vectors such as a retrovirus vector, an adenovirus vector, an adeno-associated virus vector, a vaccinia virus vector, alentivirus vector, a herpes virus vector, an alphavirus vector, an EB virus vector, a papilloma virus vector, and a foamy virus vector. Any such expression vector may include a promoter for transcribing dsRNA and DNA that encodes the dsRNA linked downstream of the promoter. In addition, a method known per se may be applied as, for example, a method of constructing the promoter or the dsRNA.

When the "substance capable of inhibiting a cell cycle-dependent RhoGAP" contained as an active ingredient in the novel antitumor agent of the present invention is a nucleic acid substance such as an antisense oligonucleotide, shRNA, siRNA, or microRNA against the gene encoding the cell cycle-dependent RhoGAP such as ARHGAP11A, the nucleic acid substance is preferably introduced into cells on which the nucleic acid substance is intended to act. As a technique for introducing the nucleic acid substance, there may be applied a method known per se such as a lipofection method, a calcium phosphate method, electroporation, or a gene gun, or a gene transfer technology to be developed, and a commercially available gene transfection reagent or kit may be utilized. In addition, in the case of, for example, a dsRNA expression vector using a viral vector retaining an infectious capacity, the vector may be incorporated into cells by means of the infectious capacity of the virus.

Among the "substances capable of inhibiting a cell cycle-dependent RhoGAP" to be contained as an active ingredient in the novel antitumor agent of the present invention, the natural polymer is exemplified by an antibody. The antibody may be a polyclonal antibody or a monoclonal antibody. The antibody may be obtained by production by a method known per se.

The novel antitumor agent of the present invention described above can markedly reduce the mobility/invasive capacity of cancer cells, thereby suppressing invasion and/or metastasis. Therefore, the present invention also encompasses a metastasis inhibitor and/or invasion inhibitor for a malignant tumor, including the "substance capable of inhibiting a cell cycle-dependent RhoGAP. "

Cancer cells to be targeted by the novel antitumor agent containing as an active ingredient a "substance capable of inhibiting a cell cycle-dependent RhoGAP" of the present invention are not particularly limited, and examples thereof may include cancer cells whose metastasis or invasion may be induced by the expression of the cell cycle-dependent RhoGAP such as ARHGAP11A. Specific examples thereof may include cancer cells derived from colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, and skin cancer, and a particularly suitable example thereof is colorectal cancer or pancreatic cancer.

Further, it is considered that the novel antitumor agent containing as an active ingredient a "substance capable of inhibiting a cell cycle-dependent RhoGAP" of the present invention can act more effectively on cancer at the T2, T3, or T4 stage (according to the TNM classification of the UICC), at which the cancer invades a tissue to reach the muscularis propria, the serosa, or the outside of the serosa, respectively. This is because in the present invention, it has been elucidated that the expression level of the RhoGAP such as ARHGAP11A serving as a therapeutic target increases in cancer at the T2, T3, or T4 stage.

The novel antitumor agent containing as an active ingredient a "substance capable of inhibiting a cell cycle-dependent RhoGAP" of the present invention may be formulated by mixing this ingredient with other ingredients as necessary. In addition, the novel antitumor agent may also be formulated by utilizing a known pharmaceutical production method. Depending on the active ingredient, the novel antitumor agent may be formulated as a nucleic acid formulation or a low-molecular-weight compound formulation. In the formulation, the novel antitumor agent may be formulated in combination with, for example, a medium or carrier to be generally used in a drug, specifically, sterile water, physiological saline, a plant oil, an emulsifier, a suspension, a surfactant, a stabilizer, or the like as appropriate. Therefore, the present invention also encompasses a therapeutic and/or prophylactic method for a malignant tumor, including using the novel antitumor agent containing as an active ingredient a "substance capable of inhibiting a cell cycle-dependent RhoGAP" of the present invention.

The present invention also encompasses a screening method for a novel antitumor agent, including selecting a "substance capable of inhibiting a cell cycle-dependent RhoGAP." Herein, a typical example of the cell cycle-dependent RhoGAP is ARHGAP11A as described above. The present invention specifically encompasses a screening method including at least the following steps A) to D). Any one of the step A) and the step B) may be performed before the other, or the steps may be simultaneously performed. Herein, a candidate substance to be selected by the screening may be, for example, a nucleic acid substance, a low-molecular-weight compound, or a natural polymer. Specific examples thereof include an antisense oligonucleotide, shRNA, siRNA, and microRNA against a gene encoding the ARHGAP11A, and a low-molecular-weight compound or natural polymer capable of binding to a transcription product or translation product of the gene. An example of the natural polymer is an antibody. The antibody may be a polyclonal antibody or a monoclonal antibody. The antibody may be obtained by production by a method known per se.
A) A step of culturing a candidate substance together with a cancer cell line, followed by measurement of an expression level of a gene encoding a given cell cycle-dependent RhoGAP (such as ARHGAP11A) in the cell;
B) a step of measuring an expression level of the gene encoding the given RhoGAP measured by the same technique as that of the step A) in a control system;
C) a step of comparing the expression levels of the gene encoding the given RhoGAP measured in the steps A) and B) to each other; and
D) a step of selecting a candidate substance in a case where the expression level of the gene measured in the step A) is lower than the expression level of the gene measured in the step B).

The step B) of the screening method refers to a step including culturing cancer cells in a system free of the candidate substance and measuring the expression level of the gene encoding the given RhoGAP measured by the same technique as that in the step A). The cancer cells that may be used in the step A) and the step B) have only to be cells capable of expressing a given RhoGAP (such as ARHGAP11A), and are not particularly limited. Examples thereof include cell lines derived from, for example, colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, and skin cancer, and a preferred example is colorectal cancer or pancreatic cancer. Particularly specifically, it is most suitable to use HCT116, which is a colorectal cancer cell line.

The expression level of the gene encoding the cell cycle-dependent RhoGAP such as ARHGAP11A may be confirmed by a technique known per se such as a real-time RT-PCR method, northern blotting, western blotting, or immunostaining or a novel technique to be developed in the future.

The present invention also encompasses a method of testing for a malignant tumor, including quantifying an expression level of a gene encoding a cell cycle-dependent RhoGAP such as ARHGAP11A in a biological specimen. An example of the cell cycle-dependent RhoGAP is ARHGAP11A. The expression level of the gene may be measured by a technique known per se such as a microarray method, a real-time RT-PCR method, northern blotting, western blotting, or immunostaining or a novel technique to be developed in the future.

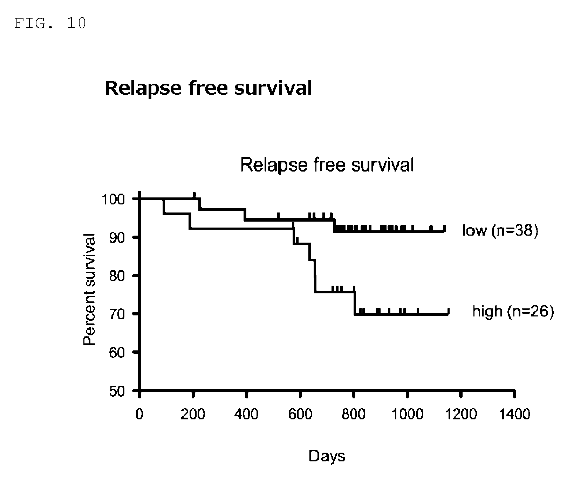

In the present invention, the biological specimen has only to be a biological specimen in which the above-mentioned protein can be detected, and is not particularly limited. Examples thereof include a wide range of biological specimens such as a tissue, blood such as plasma and serum, spinal fluid, lymph, urine, lacrimal fluid, and milk, and a suitable example of the biological specimen is a tissue. The tissue maybe acquiredbymeans of, for example, a removed diseased tissue, a biopsy, or circulating tumor cells (CTC). As shown in Examples to be described later as well, for example, when the relapse free survivals of patients who have undergone the surgical resection of colorectal cancer in a patient group with lower than average ARHGAP11A expression and a patient group with higher than average ARHGAP11A expression were confirmed, it was confirmed that the patient group with the lower expression of ARHGAP11A had a significantly lower percent relapse free survival (FIG. 10). Thus, the stage of progression or prognosis of cancer can be predicted by the method of testing of the present invention for a malignant tumor that is considered to express ARHGAP11A, such as one or more kinds selected from colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, and skin cancer.

### Examples

Hereinafter, the results of experiments performed to complete the present invention are shown as Reference Examples, and the present invention is more specifically described in Examples. However, the present invention is not limited thereto, and various applications are possible without departing from the technical concept of the present invention.

### (Reference Example 1) Confirmation of cell cycle

First, cells of a surgically excised colorectal cancer tissue classified on the basis of the presence of Geminin (cell cycle: S/G2/M stages) were stained by a tissue immunostaining method using an anti-Geminin (S/G2/M) polyclonal antibody to confirm a non-cancer portion (A), a cancer cell invasion head portion (B), and a cancer central area (C). As a result, Geminin was most intensely stained at the portion (B), confirming that the cells in the S/G2/M phases were abundantly present in the cancer cell invasion head portion (FIG. 1).

### (Reference Example 2) Analysis of motile cells

An intravital imaging experimental system for a cancer tissue was used to perform real-time analysis of the manner in which HCT116 (human colorectal cancer cell line) invaded a normal tissue. S/G2/M cells and G1 cells were sorted and subjected to microarray analysis to extract cell cycle-dependent genes, and it was found for the first time that among the genes, ARHGAP11A associated with the mobility was expressed at a high level in the cells in the S/G2/M phases.

Fluorescent probe (Fucci: fluorescent ubiquitination-based cell cycle indicator)-expressing HCT116 was implanted into the cecum or subcutaneous tissue of a NOD/SCID mouse to visualize the cell cycle progression of HCT116. Fucci caused red emission from nuclei in the G1 phase (mKO2) and green emission from nuclei in the S/G2/M phases (mAG). Part of Cdt1 protein was used in the visualization of the G1 phase, and part of Geminin protein was used in the visualization of the S/G2/Mphases. Fucci-expressing cells were separated with FACSAria II (BD Biosciences) into mAG-positive cells in the S/G2/M phases and mKO2-positive cells in the G1 phase. Constitutive expression of Fucci allows stable visualization of a cell cycle. As a result, it was confirmed that the cells in the S/G2/M phases migrated to a great extent in HCT116 (FIGS. 2).As a result of confirming cell tracking velocities, it was confirmed that the cells in the S/G2/M phases had a larger mean migration distance, thus being more mobile cells as compared to the cells in the G1 phase (FIG. 3).

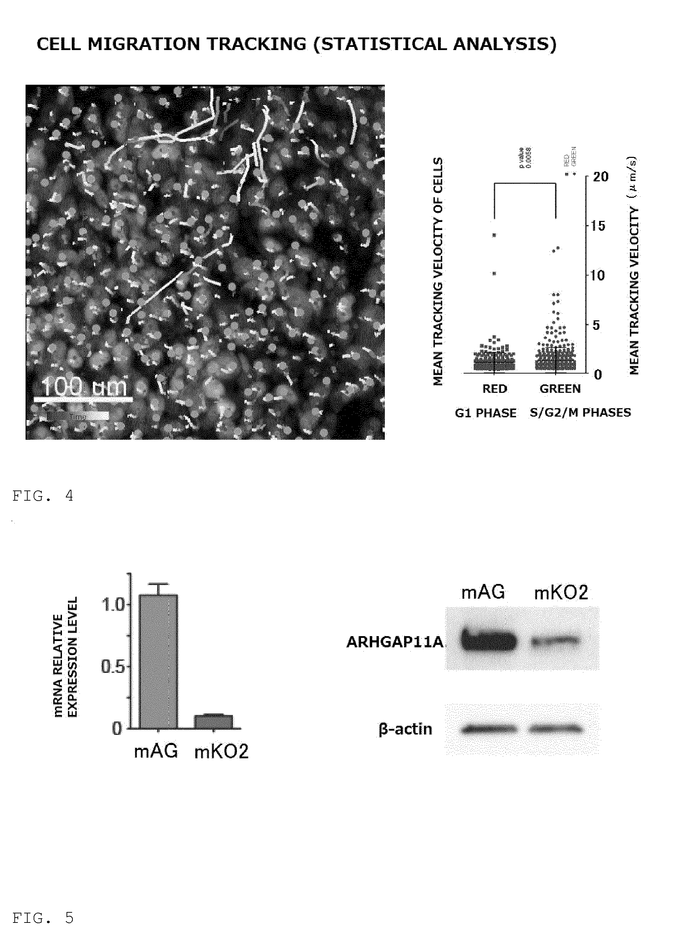

Next, a cDNA microarray was used to analyze mRNA expressed in the cells in the S/G2/M phases and the cells in the G1 phase. In the microarray analysis, 1,656 genes that were statistically significant by showing 2-fold or more increases were extracted (Table 1). As a result, it was confirmed that ARHGAP11A, a RhoGAP, was particularly intensely expressed in the cells in the S/G2/M phases at the gene level and the protein level (Table 1, FIGS. 4).

### (Reference Example 3) On expression control of ARHGAP11A by cell cycle-dependent transcription factor E2F

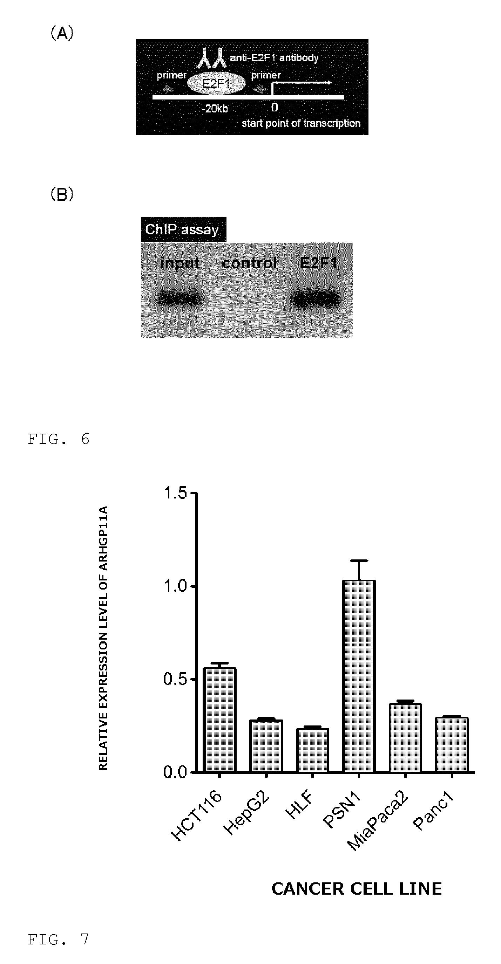

The E2F family is known to include cell cycle-dependent transcription regulators. It was noticed that there was a sequence to which E2Fs bound at -20 to -28 b (chr15: 32907663-32907671) from the start point of transcription of ARHGAP11A (chr15: 32907691), and it was confirmed by a luciferase reporter assay (FIG. 5A) and a chromatin immunoprecipitation method (ChIP) that the expression of ARHGAP11A was controlled by the cell cycle-dependent transcription factor E2F. It was confirmed that the inhibition of the expression of ARHGAP11A reduced the migratory/invasive capacity of cancer cells, indicating that ARHGAP11A was a promising therapeutic target (FIG. 5B).

**[Table 1]**

| Rho GAPs | | |
|---|---|---|
| Rank | Gene | FC |
| 5 | ARHGAP11A | 18.85 |
| 12 | ARHGAP11A | 14.90 |
| 41 | ARHGAP11A | 11.03 |
| 57 | DEPDCID | 9.84 |
| 106 | IQGAP3 | 7.47 |

### (Reference Example 4) On target diseases

In this reference example, cancer cells to be targeted by the novel antitumor agent of the present invention were confirmed. That is, it was considered that cancer cells whose metastasis or invasion might be induced by the expression of ARHGAP11A were cancer cells to be targeted by the novel antitumor agent containing as an active ingredient a substance capable of inhibiting ARHGAP11A of the present invention. For human colorectal cancer cells (HCT116), human liver cancer cells (HepG2), lymphatic fibroblasts (Human Lymphatic Fibroblasts: HLF), and human pancreatic adenocarcinoma cells (PSN1, MiaPaca2, Panc1), the expression of ARHGAP11A was confirmed by a quantitative real-time PCR method. β-Actin was used as an internal control, and the results were shown as values relative to the expression level of β-actin defined as 1. As a result, the expression of ARHGAP11A was found in cancer cells derived from the human colorectal cancer cells in which the expression of ARHGAP11A was found and the human pancreatic adenocarcinoma cells (HCT116, PSN1) (FIG. 6). Thus, it is considered that the novel antitumor agent of the present invention can act effectively on these cancer cells.

Inaddition, referring to GeneCards ID: GC15P032907 of GeneNote, microarray expression of ARHGAP11A has been found in cancer cells derived from, for example, colorectal cancer, pancreatic cancer, prostate cancer, breast cancer, head and neck cancer, melanoma, ovarian cancer, lung cancer, brain cancer, pancreatic cancer, hepatocellular carcinoma, renal cell carcinoma, and skin cancer. Accordingly, it is considered that the novel antitumor agent of the present invention can act effectively on these cancer cells. It should be noted that in GeneCards ID: GC15P032907 of GeneNote, it is only described that expression has been found in the cancer cells, and no mention is made of an influence of the expression of ARHGAP11A on the cancer cells or an effect in the case where ARHGAP11A is inhibited.

### (Example 1) Confirmation of antitumor effect based on ARHGAP11A inhibitory action

In this example, an effect of the novel antitumor agent of the present invention was confirmed by confirming the function of ARHGAP11A out of the cell cycle-dependent RhoGAPs. Lentiviral vectors containing two kinds of shRNAs (SH) having a base sequence set forth in SEQ ID NO: 1 or 2 below were produced, and were each introduced into HCT116 (human colorectal cancer cell line) to construct lines with inhibited ARHGAP11A expression (SH#1 and SH#2) . As a control, a lentiviral vector containing an oligonucleotide having a base sequence set forth in SEQ ID NO: 3 below was produced, and was introduced into HCT116 by the same technique. For each of the lines with inhibited ARHGAP11A expression (SH#1 and SH#2), cell proliferative capacity and cell invasion were confirmed.

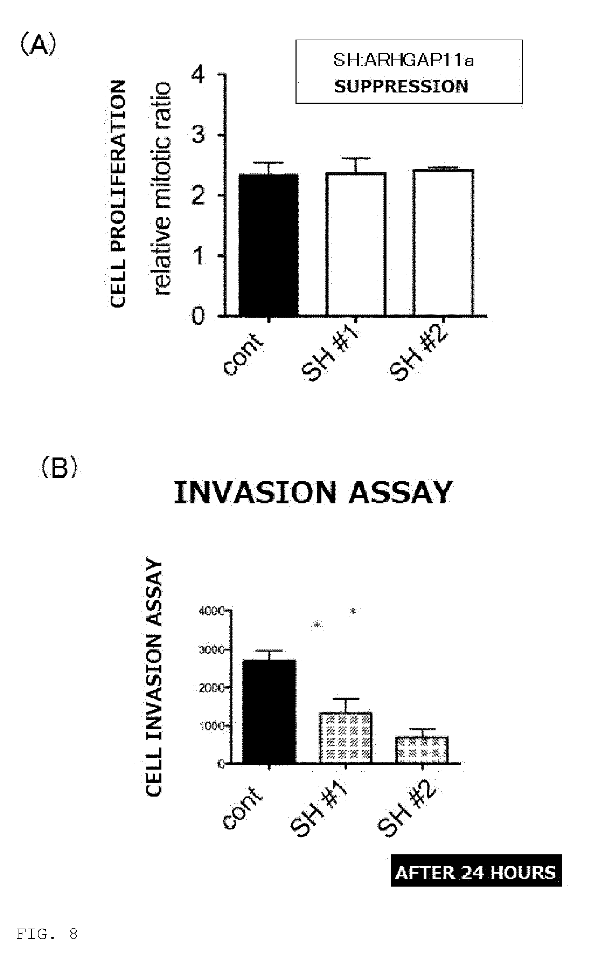

Cell proliferation was analyzed by a BrdU proliferation assay method involving adding a precursor substance (5-bromo-2'-deoxyuridine: BrdU) for label DNA to cells, and analyzing quantified incorporation thereof into genomic DNA in the S phase of the cell cycle (replication). A cancer cell invasion assay was performed using BD BioCoat™ Matrigel Invasion Chamber in accordance with the method of the instruction manual. As a result, no difference from the control was found in cell proliferative capacity (FIG. 7A). For the cell invasive capacity, the lines with inhibited ARHGAP11A expression (SH#1 and SH#2) were found to have low values as compared to the control (FIG. 7B). Thus, the suppression of invasion was observed in the lines with inhibited ARHGAP11A expression (FIGS. 7A and 7B). In view of the foregoing, an anticancer therapy involving inhibiting ARHGAP11A is considered to be a breakthrough therapeutic method by which invasion by human colorectal cancer is suppressed, and drug development is strongly expected in the future.

The DNA sequences of the shRNAs against ARHGAP11A and the control are as follows.
shRNA target: Sequence Strand (5'-3')
1) ARHGAP11A #1 (TRCN0000047281) : CCGGCGGTATCAGTTCACATCGATACTCGAGTATCGATGTGAACTGATACCGTTTTTG (SEQ ID NO: 1)
2) ARHGAP11A #2 (TRCN0000047282): CCGGCCTTCTATTACACCTCAAGAACTCGAGTTCTTGAGGTGTAATAGAAGGTTTTTG (SEQ ID NO: 2)
3) Control (SHC002): CCGGCAACAAGATGAAGAGCACCAACTCGAGTTGGTGCTCTTCATCTTGTTGTTTTT (SEQ ID NO: 3)

### (Example 2) Confirmation of antitumor effect based on ARHGAP11A inhibitory action

In this example, an *in vivo* antitumor effect based on the ARHGAP11A inhibitory action was confirmed.

1 × 10⁶ HCT116 cells of a wild-type line having introduced therein no lentiviral vector were implanted into immunocompromised mice (NOD/SCID) by local injection.

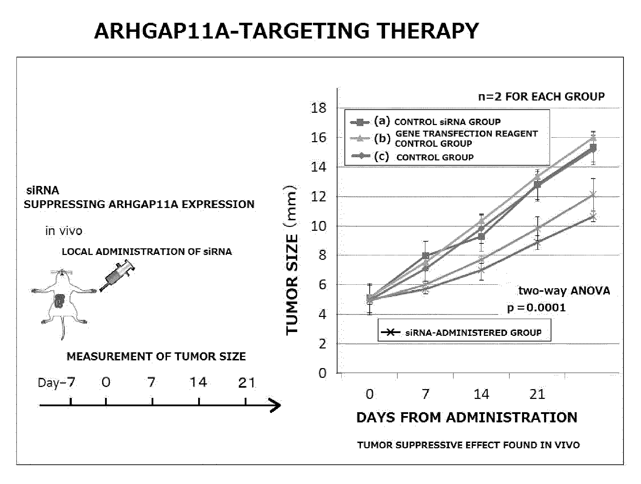

After that, the lentiviral vector containing shRNA (SH) having the base sequence set forth in SEQ ID NO: 1 or 2 (SH#1 or SH#2) produced in Example 1 was locally injected using AteloGene™ (Koken), an *in vivo* siRNA transfection kit (siRNA-administered group). As a control group, (a) the lentiviral vector containing shRNA having the base sequence set forth in SEQ ID NO: 3 produced in Example 1 (control shRNA group) or (b) a lentiviral vector 1 subjected to no genetic recombination was introduced in the same manner as above using AteloGene™ (Koken) (gene transfection reagent control group) . Further, (c) a system into which no lentivirus was locally injected (control group) was also used as a control. The expansion of the tumor cells 4 weeks after the implantation was confirmed on the basis of the magnitude of the size of the tumor. The administration of shRNA against ARHGAP11A into the tumor was able to significantly suppress the expansion of the tumor (FIG. 8).

### (Example 3) Relationship between expression of ARHGAP11A and colorectal cancer staging

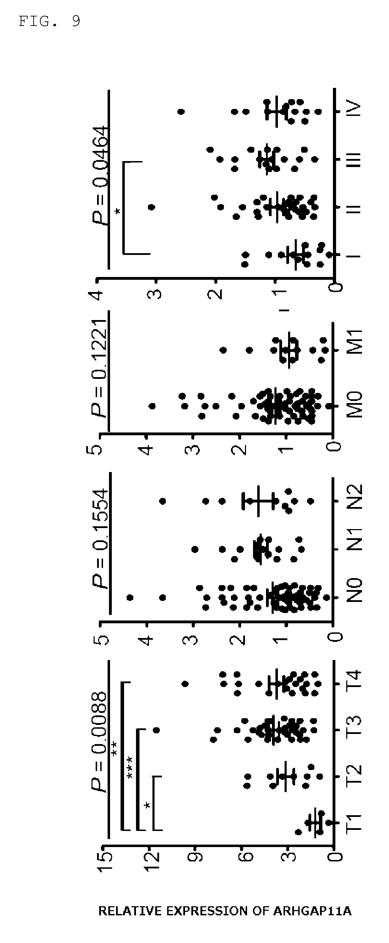

Cancer tissue portions were separated from surgical specimens of patients who had undergone the surgical resection of colorectal cancer by a laser microdissection method, mRNA was isolated therefrom, and the expression of ARHGAP11A was confirmed by a microarray method. Comparing the surgically excised specimens of colorectal cancer and normal colon mucosa, the expression of ARHGAP11A was increased at the cancer portion, and it was confirmed by colorectal cancer staging that the expression of ARHGAP11A was increased with stage progression. For example, it was revealed that the expression was increased at the T2, T3, and T4 stages as compared to the T1 (according to the TNM classification of the UICC), at which cancer invades a tissue to reach the muscularis propria (FIGS. 9).

### (Example 4) Test for prognostic prediction of cancer surgical resection

Patients who had undergone the surgical resection of colorectal cancer in the cases where cancer surgical resection (definitive surgery) was possible were classified into a high expression group and a low expression group and subjected to the analysis of relapse free survival. Cancer tissue portions were separated from surgical specimens by a laser microdissection method, mRNA was isolated therefrom, and the expression of ARHGAP11A was confirmed by a microarray method. As a result, the confirmation of relapse free survivals in the patient group with lower than average ARHGAP11A expression (n=38) and the patient group with higher than average ARHGAP11A expression (n=26) showed a significantly higher percent survival in the patient group with lower ARHGAP11A expression, confirming poor prognosis in the high expression group (FIG. 10). Thus, it is considered that cancer prognosis can be predicted by confirming the expression level of ARHGAP11A. It should be noted that the contents of Example 3 and 4 are based on the results of experiments conducted at the Medical Institute of Bioregulation at Kyushu University with the approval of an ethics committee of the university.

### (Example 5) Antisense oligonucleotide (BNA) against ARHGAP11A

In this example, suppressive effects of 35 kinds of antisense oligonucleotides each containing BNA, an artificial nucleic acid ((hereinafter referred to as "antisense BNAs") on the expression of ARHGAP11A in HCT116 (human colorectal cancer cell line) were confirmed.

First, 35 kinds of antisense BNAs having base sequences set forth in SEQ ID NOS: 4 to 38 below and oligonucleotides having base sequences set forth in SEQ ID NOS: 39 to 41 were produced. An example of ARHGAP11A is a human-derived protein synthesized from mRNA having a base sequence identified by GenBank Accession No. NM_014783.3 (variant 1) or GenBank Accession No. NM_199357.1 (variant 2). The antisense BNAs identified by SEQ ID NOS: 4 to 23 are each an antisense oligonucleotide strand against a sequence portion common to the variant 1 and the variant 2, the antisense BNAs identified by SEQ ID NOS: 24 to 33 are each an antisense oligonucleotide strand against the variant 1, and the antisense BNAs identified by SEQ ID NOS: 34 to 38 are each an antisense oligonucleotide strand against the variant 2.

In each of the sequences shown below, N(L) represents artificial nucleic acid BNA, 5 represents 5-mC (methylcytosine), 5 (L) represents L-mC (methylated artificial nucleic acid BNA), T (L) represents artificial nucleic acid thymidine, and T(A) represents artificial nucleic acid adenine. The BNAs used in this example are ones produced by GeneDesign Inc.
ARHGAP-47-BNA-16: T(L)5(L)5(L)gcccccagcT(L)5(L)5(L)t (SEQ ID NO: 4)
ARHGAP-126-BNA-16: 5 (L) T (L) 5 (L) ccccatcag5 (L) 5 (L) T (L) g (SEQ ID NO: 5)
ARHGAP-203-BNA-16: 5 (L) T (L) 5 (L) aggcaactcT (L) T (L) 5 (L) c (SEQ ID NO: 6)
ARHGAP-382-BNA-16: T (L) 5 (L) T (L) attgccagaT (L) T (L) 5 (L) t (SEQ ID NO: 7)
ARHGAP-406-BNA-16: 5 (L) T (L) T (L) tctgattctT (L) T (L) 5 (L) g (SEQ ID NO: 8)
ARHGAP-591-BNA-16: 5 (L) 5 (L) 5 (L) gtctggcacT (L) T (L) 5 (L) t (SEQ ID NO: 9)
ARHGAP-625-BNA-16: 5 (L) T (L) 5 (L) aaatttgaa5 (L) T (L) 5 (L) c (SEQ ID NO: 10)
ARHGAP-720-BNA-16: T (L) 5 (L) T (L) gatcccacaT (L) T (L) 5 (L) c (SEQ ID NO: 11)
ARHGAP-843-BNA-16: T (L) T (L) T (L) taccccctaT (L) T (L) T (L) c (SEQ ID NO: 12)
ARHGAP-969-BNA-16: T (L) 5 (L) 5 (L) gaaaaagcc5 (L) T (L) T (L) c (SEQ ID NO: 13)
ARHGAP-1006-BNA-16: T(L)T(L)5(L)tttagtgctT(L)T(L)T(L)a (SEQ ID NO: 14)
ARHGAP-1162-BNA-16: T(L)T(L)T(L)tcctctgtg5(L)5(L)T(L)a (SEQ ID NO: 15)
ARHGAP-1344-BNA-16: T(L)5(L)T(L)tttcatgtc5(L)T(L)T(L) (SEQ ID NO: 16)
ARHGAP-1447-BNA-16: T(L)5(L)5(L)aggataaaaT(L)5(L)T(L)g (SEQ ID NO: 17)
ARHGAP-1538-BNA-16: T(L)T(L)5(L)accaggagtT(L)T(L)5(L)a (SEQ ID NO: 18)
ARHGAP-1748-BNA-16: 5(L)T(L)T(L)gatggactt5(L)5(L)T(L)t (SEQ ID NO: 19)
ARHGAP-1849-BNA-16: 5(L)T(L)5(L)tgagatgac5(L)5(L)T(L)t (SEQ ID NO: 20)
ARHGAP-1931-BNA-16: T(L)T(L)T(L)gcctgcaatT(L)5(L)T(L)t (SEQ ID NO: 21)
ARHGAP-2032-BNA-16: 5(L)5(L)T(L)agattgaatT(L)T(L)5(L)a (SEQ ID NO: 22)
ARHGAP-2176-BNA-16: T(L)T(L)T(L)tcatcaacaT(L)5(L)T(L)g (SEQ ID NO: 23)
ARHGAPv1-2262-BNA-16: T(L)5(L)5(L)ggtaatttgT(L)T(L)5(L)c (SEQ ID NO: 24)
ARHGAPv1-2311-BNA-16: T(L)T(L)T(L)gcatctactT(L)5(L)T(L)t (SEQ ID NO: 25)
ARHGAPv1-2628-BNA-16: 5(L)5(L)T(L)ctgggctat5(L)T(L)T(L)c (SEQ ID NO: 26)
ARHGAPv1-2808-BNA-16: T(L)T(L)T(L)catgttccaT(L)5(L)T(L)t (SEQ ID NO: 27)
ARHGAPv1-2942-BNA-16: T(L)T(L)5(L)catcatatt5(L)T(L)5(L)a (SEQ ID NO: 28)
ARHGAPv1-3278-BNA-16: 5(L)5(L)5(L)tgtaggttgT(L)5(L)T(L)g (SEQ ID NO: 29)
ARHGAPv1-3484-BNA-16: T(L)T(L)5(L)gagggtaacT(L)5(L)5(L)a (SEQ ID NO: 30)
ARHGAPv1-4399-BNA-16: 5(L)T(L)T(L)gctccattcT(L)T(L)T(L)c (SEQ ID NO: 31)
ARHGAPv1-5140-BNA-16: 5(L)T(L)T(L)cctctacaa5(L)5(L)T(L)a (SEQ ID NO: 32)
ARHGAPv1-5194-BNA-16: T(L)5(L)T(L)aacaaccaaT(L)5(L)T(L) (SEQ ID NO: 33)
ARHGAPv2-2215-BNA-16: 5(L)T(L)5(L)taacagtagT(L)A(L)T(L)g (SEQ ID NO: 34)
ARHGAPv2-2285-BNA-16: T(L)5(L)T(L)agaacagtaA(L)A(L)T(L)t (SEQ ID NO: 35)
ARHGAPv2-2306-BNA-16: T(L)T(L)5(L)aaacatgaa5(L)T(L)T(L)t (SEQ ID NO: 36)
ARHGAPv2-2355-BNA-16: T(L)5(L)5(L)caattgttgA(L)T(L)A(L)g (SEQ ID NO: 37)
ARHGAPv2-2404-BNA-16: T(L)T(L)T(L)taacataagA(L)A(L)T(L)g (SEQ ID NO: 38)

ARHGAP-1931-BNA-16-cont1:
T(L)tT(L)gccT(L)gcaaT(L)t5(L)T(L)t (SEQ ID NO: 39)
ARHGAP-1931-BNA-16-cont2: T(L)tgT(L)ccT(L)gcT(L)aat5(L)T(L)t (SEQ ID NO: 40)
ARHGAP-1931-BNA-16-cont3: T(L)5(L)T(L)caatgcctgT(L)T(L)T(L)t (SEQ ID NO: 41)

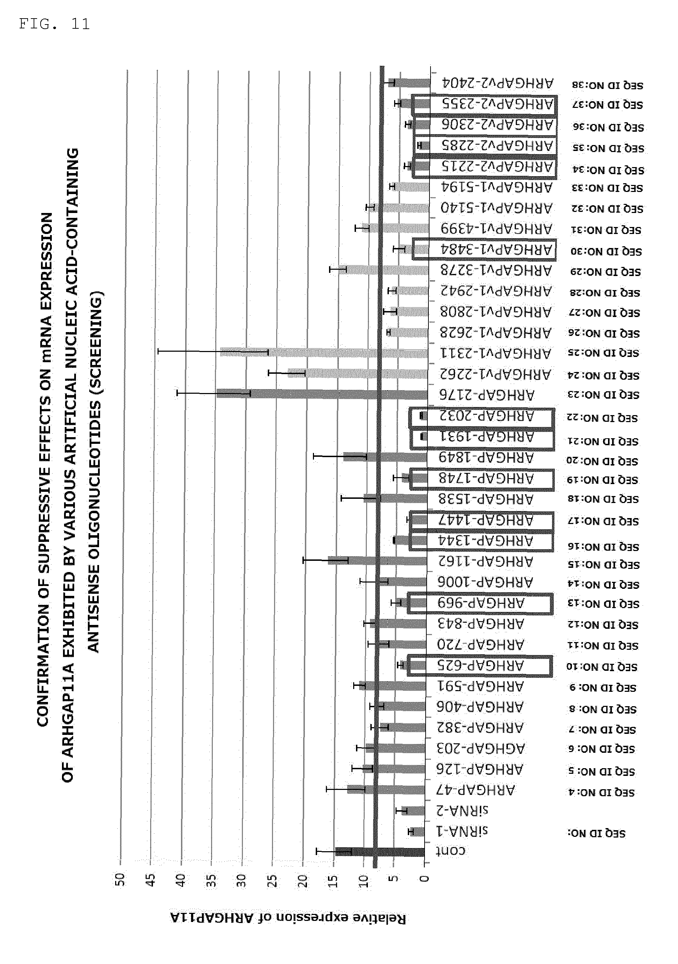

The antisense BNAs having the respective sequences shown above were each introduced into HCT116 using Lipofectamine reagent, and antisense BNAs capable of suppressing the mRNA expression of ARHGAP11A were confirmed by a qPCR method. GAPDH was used as an internal control, and the results were shown as values relative to the expression level of GAPDH defined as 1. As a result, it was confirmed that the antisense BNAs set forth in SEQ ID NOS: 10, 13, 16, 17, 19, 21, 22, 30, 34, and 35 to 38 effectively suppressed the expression of ARHGAP11A (FIG. 11).

### (Example 6) Confirmation of suppressive effect on expression of ARHGAP11A exhibited by artificial nucleic acid-containing antisense BNA

In this example, suppressive effects on the expression of ARHGAP11A in various cancer cells were confirmed for the antisense BNAs found in Example 5 to have suppressive effects on the expression of ARHGAP11A.

As the antisense BNAs, ARHGAP-625-BNA-16 (#625: SEQ ID NO: 10), ARHGAP-969-BNA-16 (#969: SEQ ID NO: 13), ARHGAP-1344-BNA-16 (#1344: SEQ ID NO: 16), ARHGAP-1447-BNA-16 (#1447: SEQ ID NO: 17), ARHGAP-1748-BNA-16 (#1748: SEQ ID NO: 19), ARHGAP-1931-BNA-16 (#1931: SEQ ID NO: 21), and ARHGAP-2032-BNA-16 (#2032: SEQ ID NO: 22) were used. As a control, ARHGAP-1931-BNA-16-cont2 set forth in SEQ ID NO: 40 was used. In addition, cancer cells to which no antisense BNA or control oligonucleotide was added were defined as wild type (wt). The suppressive effects were confirmed for the following four kinds of cancer cells: DLD1 (human colon adenocarcinoma cell line), HT29 (human colon adenocarcinoma cell line), Panc1 (human pancreatic adenocarcinoma cell line), and PSN1 (human pancreatic adenocarcinoma).

The antisense BNAs were each introduced into each kind of cancer cells by the same technique as the method described in Example 5 using Lipofectamine reagent, and their suppressive effects on the expression of ARHGAP11A were confirmed by western blotting.

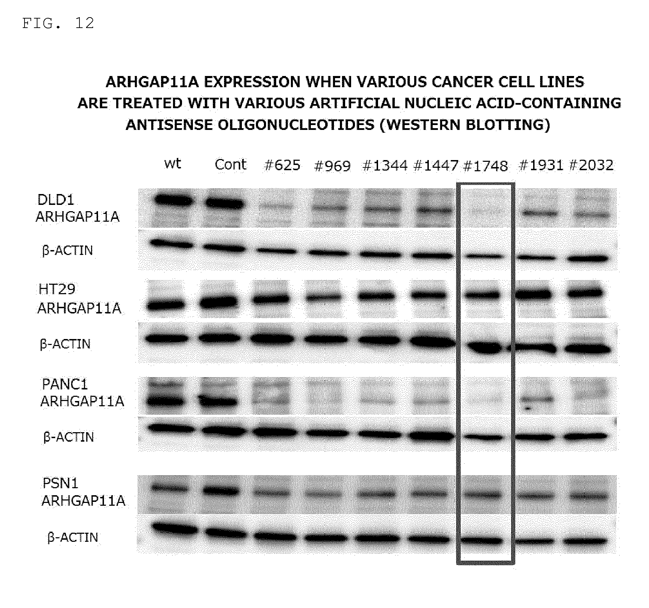

As a result, in each kind of cells, the suppression of the expression of ARHGAP11A by antisense BNA was found, and particularly strong expression suppression was found in the case of ARHGAP-1748-BNA-16 (#1748: SEQ ID NO: 19) (FIGS. 12).

### (Example 7) Confirmation of incorporation amount of antisense BNA into cells (in vitro)

In this example, the incorporation amount of FITC-labeled antisense BNA (ARHGAP-1748-BNA-16: #1748) into HCT116 was confirmed using Lipofectamine reagent. As a control, the oligonucleotide having the base sequence set forth in SEQ ID NO: 40 produced in Example 1 was used.

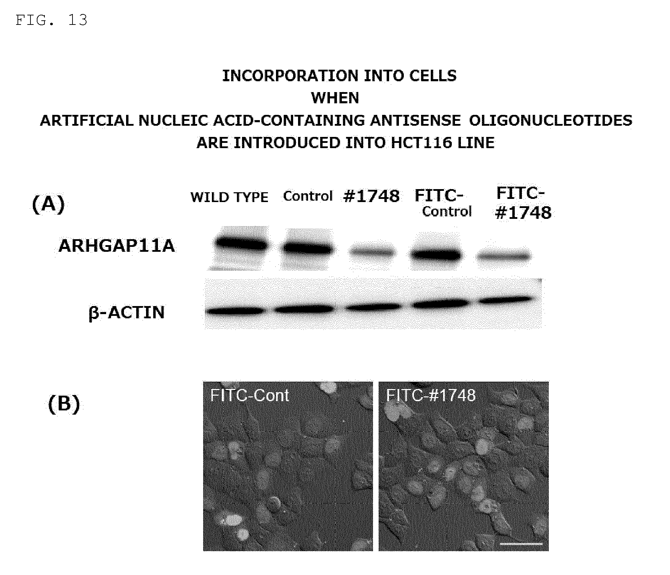

As a result, it was confirmed that the expression of ARHGAP11A was suppressed in the cells into which ARHGAP-1748-BNA-16 had been introduced (FIGS. 13(A)). In addition, the incorporation amount of ARHGAP-1748-BNA-16 into the cells was comparable to that of the control (FIGS. 13(B)).

### (Example 8) Confirmation of tumor cell proliferation suppressive effect of antisense BNA (in vivo)

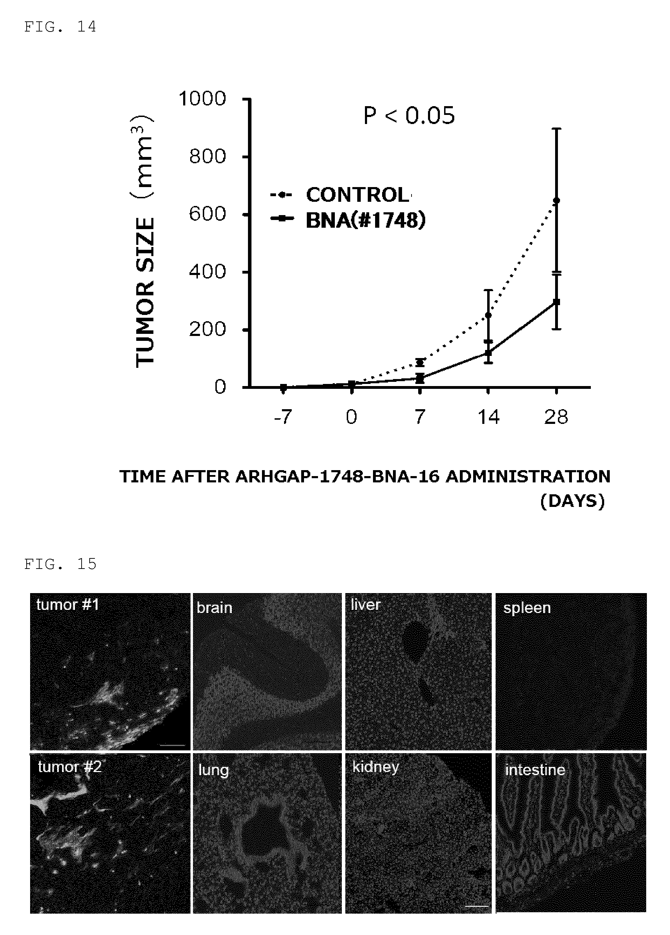

In this example, an *in vivo* antitumor effect of antisense BNA (ARHGAP-1748-BNA-16: #1748) was confirmed. HCT116 of a wild-type line was implanted into immunocompromised mice (NOD/SCID) by the same technique as that of Example 2, and then FITC-labeled ARHGAP-1748-BNA-16 was locally injected around the tumor by the same technique as that of Example 2 using AteloGene™ (Koken). It was confirmed that the administration of ARHGAP-1748-BNA-16 significantly suppressed the expansion of the tumor (FIG. 14). Further, on day 14 after the administration of ARHGAP-1748-BNA-16, incorporation of FITC-labeled ARHGAP-1748-BNA-16 into various organs (brain, liver, spleen, lung, kidney, and intestine) and tumor portion of the mice was confirmed. As a result, it was confirmed that ARHGAP-1748-BNA-16 reached only the tumor portion and did not reach the other organs (FIGS. 15).

### (Example 9) Confirmation of tumor metastasis suppressive effect of antisense BNA (in vivo)

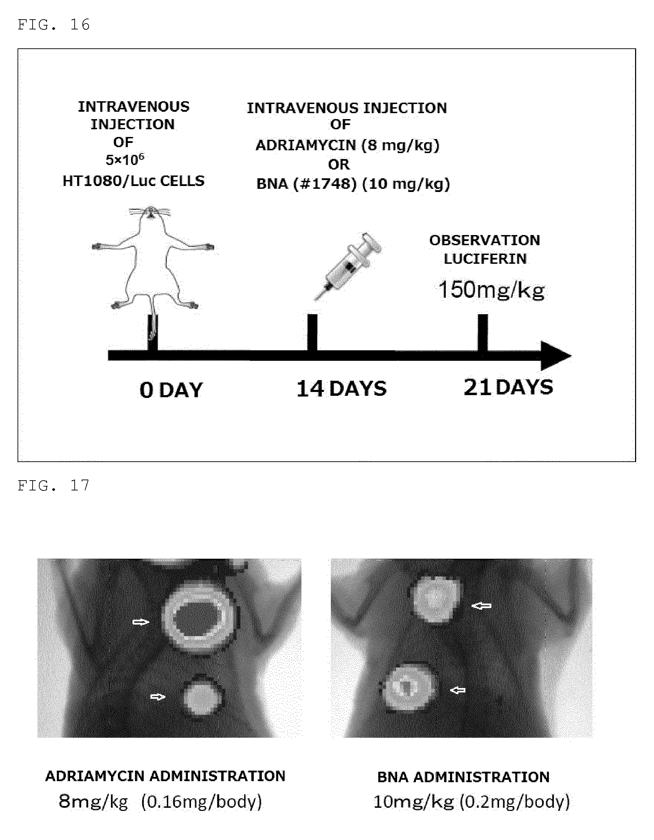

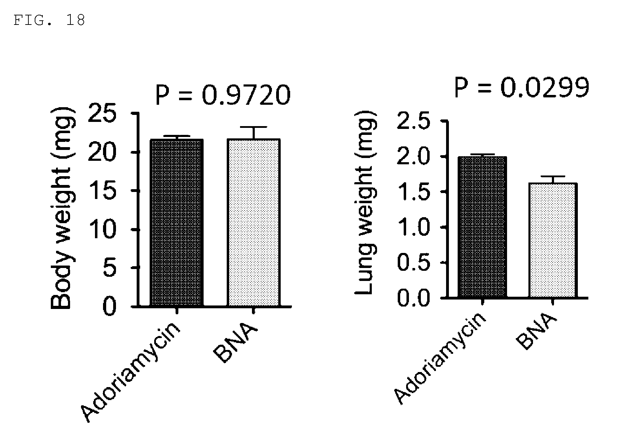

In this example, an *in vivo* metastasis suppressive effect of antisense BNA (ARHGAP-1748-BNA-16: #1748) on malignant tumor cells was confirmed. A lentivirus containing a luciferase-encoding gene was produced, and allowed to infect HT1080 (human fibrosarcoma cells) to introduce the luciferase gene. Thus, cells having introduced therein luciferase (HT1080/Luc) were produced. 5×10⁶ HT1080/Luc cells were intravenously injected into nude mice. 14 days after the injection of HT1080/Luc, 8 mg/kg of Adriamycin or 10 mg/kg of the antisense BNA were intravenously injected. After an additional 7 days, 150 mg/kg of luciferin were administered, and lung metastasis of HT1080/Luc was confirmed by luminescence imaging (FIG. 16). As a result, the tumor was smaller in the antisense BNA-administered group (n=3) than in the Adriamycin-administered group (n=3) (FIGS. 17). In addition, the weight of the lung of each of the mice was measured, and the results showed that the weight of the lung was smaller in the antisense BNA-administered group than in the Adriamycin-administered group. On the other hand, a difference in body weight was not found between the two groups (FIGS. 18). This suggested that tumor cell metastasis was suppressed to a greater degree in the antisense BNA-administered group than in the Adriamycin-administered group.

### Industrial Applicability

As described in detail above, the novel antitumor agent containing as an active ingredient a substance capable of inhibiting a cell cycle-dependent RhoGAP of the present invention suppresses cancer cell invasion and/or metastasis in, for example, cancer cells derived from cancer capable of expressing the cell cycle-dependent RhoGAP, specifically, cancer capable of expressing ARHGAP11A, such as colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, or skin cancer. Hitherto, there has been almost no effective drug that is an antitumor agent capable of suppressing cancer cell invasion and/or metastasis. The provision of the drug capable of suppressing cancer cell invasion and/or metastasis affecting malignant progression of cancer enables an effective cancer therapy. Accordingly, the present invention is useful.

Further, as a result of testing the expression of the cell cycle-dependent RhoGAP such as ARHGAP11A in a biological specimen on the basis of, for example, mRNA, significant differences have been found in association with the stage of cancer progression (staging) and relapse free survival. Thus, a test for a malignant tumor can be performed by quantifying the cell cycle-dependent RhoGAP in a biological specimen. Further, in terms of the expression of the cell cycle-dependent RhoGAP such as the mRNA level of ARHGAP11A in the biological specimen, significant differences are found in association with the stage of malignant tumor progression (staging) and relapse free survival. Thus, it is considered that the stage of progression and/or prognosis of cancer can be predicted by measuring the expression of the cell cycle-dependent RhoGAP such as ARHGAP11A in the biological specimen. Accordingly, the present invention is useful.

## Claims

1. A novel antitumor agent, comprising as an active ingredient a substance capable of inhibiting a cell cycle-dependent Rho GTPase activating protein.

2. A novel antitumor agent according to claim 1, wherein the substance capable of inhibiting a cell cycle-dependent Rho GTPase activating protein comprises a substance capable of inhibiting ARHGAP11A.

3. A novel antitumor agent according to claim 2, wherein the substance capable of inhibiting ARHGAP11A comprises any one selected from:
an antisense oligonucleotide against a gene encoding the ARHGAP11A;
an oligonucleotide that induces RNA interference of the gene encoding the ARHGAP11A; and a low-molecular-weight compound and a natural polymer each capable of binding to a transcription product or
translation product of the gene encoding the ARHGAP11A.

4. A novel antitumor agent according to claim 3, wherein the substance capable of inhibiting ARHGAP11A comprises the antisense oligonucleotide against the gene encoding the ARHGAP11A, the oligonucleotide comprising from 14 bases to 200 bases, the base sequence comprising at least one or more artificial nucleic acids.

5. A novel antitumor agent according to claim 4, wherein the antisense oligonucleotide comprises an oligonucleotide comprising a base sequence set forth in any one of the following items 1) to 13):
1) ARHGAP-625-BNA-16: 5(L)T(L)5(L)aaatttgaa5(L)T(L)5(L)c (SEQ ID NO: 10);
2) ARHGAP-969-BNA-16: T(L)5(L)5(L)gaaaaagcc5(L)T(L)T(L)c (SEQ ID NO: 13);
3) ARHGAP-1344-BNA-16: T(L)5(L)T(L)tttcatgtc5(L)T(L)T(L)c (SEQ ID NO: 16);
4) ARHGAP-1447-BNA-16: T(L)5(L)5(L)aggataaaaT(L)5(L)T(L)g (SEQ ID NO: 17);
5) ARHGAP-1748-BNA-16: 5(L)T(L)T(L)gatggactt5(L)5(L)T(L)t (SEQ ID NO: 19);
6) ARHGAP-1931-BNA-16: T(L)T(L)T(L)gcctgcaatT(L)5(L)T(L)t (SEQ ID NO: 21);
7) ARHGAP-2032-BNA-16: 5(L)5(L)T(L)agattgaatT(L)T(L)5(L)a (SEQ ID NO: 22);
8) ARHGAPv1-3484-BNA-16: T(L)T(L)5(L)gagggtaacT(L)5(L)5(L)a (SEQ ID NO: 30);
9) ARHGAPv2-2215-BNA-16: 5(L)T(L)5(L)taacagtagT(L)A(L)T(L)g (SEQ ID NO: 34);
10) ARHGAPv2-2285-BNA-16: T(L)5(L)T(L)agaacagtaA(L)A(L)T(L)t (SEQ ID NO: 35);
11) ARHGAPv2-2306-BNA-16: T(L)T(L)5(L)aaacatgaa5(L)T(L)T(L)t (SEQ ID NO: 36);
12) ARHGAPv2-2355-BNA-16: T(L)5(L)5(L)caattgttgA(L)T(L)A(L)g (SEQ ID NO: 37); and
13) ARHGAPv2-2404-BNA-16: T(L)T(L)T(L)taacataagA(L)A(L)T(L)g (SEQ ID NO: 38),
where N (L) represents artificial nucleic acid BNA, 5 (L) represents L-mC (methylated artificial nucleic acid BNA), T(L) represents artificial nucleic acid thymidine, and A (L) represents artificial nucleic acid adenine.

6. A novel antitumor agent according to claim 3, wherein the substance capable of inhibiting ARHGAP11A comprises the oligonucleotide that induces RNA interference, the oligonucleotide comprising a base sequence set forth in the following item 14) or 15):
14) ARHGAP11A #1 (TRCN0000047281): CCGGCGGTATCAGTTCACATCGATACTCGAGTATCGATGTGAACTGATACCGTTTTTG (SEQ ID NO: 1); or
15) ARHGAP11A #2 (TRCN0000047282): CCGGCCTTCTATTACACCTCAAGAACTCGAGTTCTTGAGGTGTAATAGAAGGTTTTTG (SEQ ID NO: 2).

7. A novel antitumor agent according to any one of claims 1 to 6, wherein the antitumor agent has a metastasis inhibitory action on a malignant tumor and/or an invasion inhibitory action on a malignant tumor.

8. A screening method for a novel antitumor agent, comprising selecting a substance capable of inhibiting a cell cycle-dependent Rho GTPase activating protein.

9. A screening method according to claim 8, wherein the cell cycle-dependent Rho GTPase activating protein comprises ARHGAP11A.

10. A screening method according to claim 9, comprising at least the following steps A) to D):
A) a step of culturing a candidate substance together with a cancer cell line, followed by measurement of an expression level of a gene encoding the ARHGAP11A in the cell;
B) a step of measuring an expression level of the gene encoding the ARHGAP11A measured by the same technique as that of the step A) in a control system;
C) a step of comparing the expression levels of the gene encoding the ARHGAP11A measured in the steps A) and B) to each other; and
D) a step of selecting a candidate substance in a case where the expression level of the gene measured in the step A) is lower than the expression level of the gene measured in the step B).

11. A method of testing for a malignant tumor, comprising quantifying a cell cycle-dependent Rho GTPase activating protein in a biological specimen.

12. A method of testing for a malignant tumor according to claim 11, wherein the cell cycle-dependent Rho GTPase activating protein comprises ARHGAP11A.

13. A method of testing for a malignant tumor according to claim 11 or 12, wherein the malignant tumor comprises one or more kinds selected from colorectal cancer, pancreatic cancer, prostate cancer cells, breast cancer, head and neck cancer, melanoma, ovarian cancer, lung cancer, brain cancer, pancreatic cancer, hepatocellular carcinoma, renal cell carcinoma, and skin cancer.

14. A method of testing for a malignant tumor according to any one of claims 11 to 13, wherein the testing for the malignant tumor comprises a test for predicting a stage of progression and/or prognosis of cancer.


SEQUENCE LISTING

<110>  Osaka University

<120>  Novel antitumor agent and the screening method for selecting the
antitumor agent

<130>  GP12-1024

<150>  JP P2012-15982
<151>  2012-01-27

<160>  41

<170>  PatentIn version 3.1

<210>  1
<211>  58
<212>  DNA
<213>  Artificial

<220>
<223>  shRNA ARHGAP #1

<400>  1
ccggcggtat cagttcacat cgatactcga gtatcgatgt gaactgatac cgtttttg        58


<210>  2
<211>  58
<212>  DNA
<213>  Artificial

<220>
<223>  shRNA ARHGAP #2

<400>  2
ccggccttct attacacctc aagaactcga gttcttgagg tgtaatagaa ggtttttg        58


<210>  3
<211>  57
<212>  DNA
<213>  Artificial

<220>
<223>  shRNA Control

<400>  3
ccggcaacaa gatgaagagc accaactcga gttggtgctc ttcatcttgt tgttttt         57


<210>  4
<211>  16
<212>  DNA
<213>  Artificial

<220>
<223>  Antisense oligonucleotide: ARHGAP-47-BNA-16

<220>
<221>  misc_feature
<222>  (13)..(15)

<223>  BNA, n=5-mC

<220>
<221>  misc_feature
<222>  (1)..(3)
<223>  BNA, n=5-mC

<400>  4
tnngccccca gctnnt                                                        16

<210>  5
<211>  16
<212>  DNA
<213>  Artificial

<220>
<223>  Antisense oligonucleotide: ARHGAP-126-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  5
ntnccccatc agnntg                                                        16

<210>  6
<211>  16
<212>  DNA
<213>  Artificial

<220>
<223>  Antisense oligonucleotide: ARHGAP-203-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  6
ntnaggcaac tcttnc                                                        16

<210>  7
<211>  16

<212>  DNA
<213>  Artificial

<220>
<223>  Antisense oligonucleotide: ARHGAP-382-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  7
tntattgcca gattnt                                                       16


<210>  8
<211>  16
<212>  DNA
<213>  Artificial

<220>
<223>  Antisense oligonucleotide: ARHGAP-406-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  8
ntttctgatt ctttng                                                       16


<210>  9
<211>  16
<212>  DNA
<213>  Artificial

<220>
<223>  Antisense oligonucleotide: ARHGAP-591-BNA-16

<220>
<221>  misc_feature
<222>  (1)..(3)
<223>  BNA, n=5-mC


<220>
<221>  misc_feature
<222>  (13)..(15)

<223>  BNA, n=5-mC

<400>  9
nnngtctggc acttnt                                                       16

<210>  10
<211>  16
<212>  DNA
<213>  Artificial

<220>
<223>  Antisense oligonucleotide: ARHGAP-625-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  10
ntnaaatttg aantnc                                                       16

<210>  11
<211>  16
<212>  DNA
<213>  Artificial

<220>
<223>  Antisense oligonucleotide: ARHGAP-720-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  11
tntgatccca cattnc                                                       16

<210>  12
<211>  16
<212>  DNA
<213>  Artificial

<220>
<223>  Antisense oligonucleotide: ARHGAP-843-BNA-16

<220>
<221>  misc_feature
<222>  (1)..(3)
<223>  BNA


<220>
<221>  misc_feature
<222>  (13)..(15)
<223>  BNA


<400>  12
ttttaccccc tatttc                                                       16


<210>  13
<211>  16
<212>  DNA
<213>  Artificial

<220>
<223>  Antisense oligonucleotide: ARHGAP-969-BNA-16

<220>
<221>  misc_feature
<222>  (13)..(15)
<223>  BNA


<220>
<221>  misc_feature
<222>  (1)..(3)
<223>  BNA, n=5-mC


<400>  13
tnngaaaaag ccnttc                                                       16


<210>  14
<211>  16
<212>  DNA
<213>  Artificial

<220>
<223>  Antisense oligonucleotide: ARHGAP-1006-BNA-16

<220>
<221>  misc_feature
<222>  (1)..(3)
<223>  BNA, n=5-mC


<220>
<221>  misc_feature
<222>  (13)..(15)
<223>  BNA


<400>  14
ttntttagtg ctttta                                                       16

<210>  15
<211>  16
<212>  DNA
<213>  Artificial

<220>
<223>  Antisense oligonucleotide: ARHGAP-1162-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  15
ttttcctctg tgnnta                                                       16


<210>  16
<211>  15
<212>  DNA
<213>  Artificial

<220>
<223>  Antisense oligonucleotide: ARHGAP-1344-BNA-16

<220>
<221>  misc_feature
<222>  (13)..(15)
<223>  BNA, n=5-mC


<220>
<221>  misc_feature
<222>  (1)..(3)
<223>  BNA, n=5-mC


<400>  16
tnttttcatg tcntt                                                        15


<210>  17
<211>  16
<212>  DNA
<213>  Artificial

<220>
<223>  Antisense oligonucleotide: ARHGAP-1447-BNA-16

<220>
<221>  misc_feature
<222>  (1)..(3)
<223>  BNA, n=5-mC

<220>
<221>  misc_feature
<222>  (13)..(15)
<223>  BNA, n=5-mC


<400>  17
tnnaggataa aatntg                                                       16


<210>  18
<211>  16
<212>  DNA
<213>  Artificial

<220>
<223>  Antisense oligonucleotide: ARHGAP-1538-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  18
ttnaccagga gtttna                                                       16


<210>  19
<211>  16
<212>  DNA
<213>  Artificial

<220>
<223>  Antisense oligonucleotide: ARHGAP-1748-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  19
nttgatggac ttnntt                                                       16


<210>  20
<211>  16
<212>  DNA
<213>  Artificial

<220>
<223>  Antisense oligonucleotide: ARHGAP-1849-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  20
ntntgagatg acnntt                                                       16


<210>  21
<211>  16
<212>  DNA
<213>  Artificial

<220>
<223>  Antisense oligonucleotide: ARHGAP-1931-BNA-16

<220>
<221>  misc_feature
<222>  (1)..(3)
<223>  BNA


<220>
<221>  misc_feature
<222>  (13)..(15)
<223>  BNA, n=5-mC


<400>  21
tttgcctgca attntt                                                       16


<210>  22
<211>  16
<212>  DNA
<213>  Artificial

<220>
<223>  Antisense oligonucleotide: ARHGAP-2032-BNA-16

<220>
<221>  misc_feature
<222>  (1)..(3)
<223>  BNA, n=5-mC


<220>
<221>  misc_feature
<222>  (13)..(15)
<223>  BNA, n=5-mC

<400>  22
nntagattga atttna                                                       16


<210>  23
<211>  16
<212>  DNA
<213>  Artificial

<220>
<223>  Antisense oligonucleotide: ARHGAP-2176-BNA-16

<220>
<221>  misc_feature
<222>  (1)..(3)
<223>  BNA


<220>
<221>  misc_feature
<222>  (13)..(15)
<223>  BNA, n=5-mC


<400>  23
ttttcatcaa catntg                                                       16


<210>  24
<211>  16
<212>  DNA
<213>  Artificial

<220>
<223>  Antisense oligonucleotide: ARHGAPv1-2262-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  24
tnnggtaatt tgttnc                                                       16


<210>  25
<211>  16
<212>  DNA
<213>  Artificial

<220>
<223>  Antisense oligonucleotide: ARHGAPv1-2311-BNA-16

<220>
<221>  misc_feature
<222>  (1)..(3)

<223>  BNA

<220>
<221>  misc_feature
<222>  (13)..(15)
<223>  BNA, n=5-mC

<400>  25
tttgcatcta cttntt                                                    16

<210>  26
<211>  16
<212>  DNA
<213>  Artificial

<220>
<223>  Antisense oligonucleotide: ARHGAPv1-2628-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  26
nntctgggct atnttc                                                    16

<210>  27
<211>  16
<212>  DNA
<213>  Artificial

<220>
<223>  Antisense oligonucleotide: ARHGAPv1-2808-BNA-16

<220>
<221>  misc_feature
<222>  (1)..(3)
<223>  BNA

<220>
<221>  misc_feature
<222>  (13)..(15)
<223>  BNA, n=5-mC

<400>  27
tttcatgttc catntt                                                    16

<210>  28
<211>  16

<212>  DNA
<213>  Artificial

<220>
<223>  Antisense oligonucleotide: ARHGAPv1-2942-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  28
ttncatcata ttntna                                                    16


<210>  29
<211>  16
<212>  DNA
<213>  Artificial

<220>
<223>  Antisense oligonucleotide: ARHGAPv1-3278-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  29
nnntgtaggt tgtntg                                                    16


<210>  30
<211>  16
<212>  DNA
<213>  Artificial

<220>
<223>  Antisense oligonucleotide: ARHGAPv1-3484-BNA-16

<220>
<221>  misc_feature
<222>  (1)..(3)
<223>  BNA, n=5-mC


<220>
<221>  misc_feature
<222>  (13)..(15)

<223>  BNA, n=5-mC

<400>  30
ttngagggta actnna                                                        16

<210>  31
<211>  16
<212>  DNA
<213>  Artificial

<220>
<223>  Antisense oligonucleotide: ARHGAPv1-4399-BNA-16

<220>
<221>  misc_feature
<222>  (1)..(3)
<223>  BNA

<220>
<221>  misc_feature
<222>  (13)..(15)
<223>  BNA, n=5-mC

<400>  31
nttgctccat tctttc                                                        16

<210>  32
<211>  16
<212>  DNA
<213>  Artificial

<220>
<223>  Antisense oligonucleotide: ARHGAPv1-5140-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  32
nttcctctac aannta                                                        16

<210>  33
<211>  15
<212>  DNA
<213>  Artificial

<220>
<223>  Antisense oligonucleotide: ARHGAPv1-5194-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  33
tntaacaacc aatnt                                                        15


<210>  34
<211>  16
<212>  DNA
<213>  Artificial

<220>
<223>  Antisense oligonucleotide: ARHGAPv2-2215-BNA-16

<220>
<221>  misc_feature
<222>  (1)..(3)
<223>  BNA, n=5-mC


<220>
<221>  misc_feature
<222>  (13)..(15)
<223>  BNA


<400>  34
ntntaacagt agtatg                                                       16


<210>  35
<211>  16
<212>  DNA
<213>  Artificial

<220>
<223>  Antisense oligonucleotide: ARHGAPv2-2285-BNA-16

<220>
<221>  misc_feature
<222>  (1)..(3)
<223>  BNA, n=5-mC


<220>
<221>  misc_feature
<222>  (13)..(15)
<223>  BNA


<400>  35
tntagaacag taaatt                                                       16

<210>  36
<211>  16
<212>  DNA
<213>  Artificial

<220>
<223>  Antisense oligonucleotide: ARHGAPv2-2306-BNA-16

<220>
<221>  misc_feature
<222>  (1)..(3)
<223>  BNA, n=5-mC


<220>
<221>  misc_feature
<222>  (13)..(15)
<223>  BNA, n=5-mC


<400>  36
ttnaaacatg aanttt                                                    16


<210>  37
<211>  16
<212>  DNA
<213>  Artificial

<220>
<223>  Antisense oligonucleotide: ARHGAPv2-2355-BNA-16

<220>
<221>  misc_feature
<222>  (1)..(3)
<223>  BNA, n=5-mC


<220>
<221>  misc_feature
<222>  (13)..(15)
<223>  BNA


<400>  37
tnncaattgt tgatag                                                    16


<210>  38
<211>  16
<212>  DNA
<213>  Artificial

<220>
<223>  Antisense oligonucleotide: ARHGAPv2-2404-BNA-16

<220>
<221>  misc_feature
<222>  (1)..(3)
<223>  BNA

<220>
<221>  misc_feature
<222>  (13)..(15)
<223>  BNA

<400>  38
ttttaacata agaatg                                                    16

<210>  39
<211>  16
<212>  DNA
<213>  Artificial

<220>
<223>  Antisense oligonucleotide: ARHGAP-1931-BNA-16-cont1

<220>
<221>  misc_feature
<222>  (1)..(1)
<223>  BNA

<220>
<221>  misc_feature
<222>  (3)..(3)
<223>  BNA

<220>
<221>  misc_feature
<222>  (7)..(7)
<223>  BNA

<220>
<221>  misc_feature
<222>  (12)..(12)
<223>  BNA, n=5-mC

<220>
<221>  misc_feature
<222>  (14)..(14)
<223>  BNA, n=5-mC

<220>
<221>  misc_feature
<222>  (15)..(15)
<223>  BNA

<400>  39
tttgcctgca attntt                                                    16

<210>  40
<211>  16
<212>  DNA
<213>  Artificial

<220>
<223>  Antisense oligonucleotide: ARHGAP-1931-BNA-16-cont2

<220>
<221>  misc_feature
<222>  (1)..(1)
<223>  BNA


<220>
<221>  misc_feature
<222>  (4)..(4)
<223>  BNA


<220>
<221>  misc_feature
<222>  (7)..(7)
<223>  BNA


<220>
<221>  misc_feature
<222>  (10)..(10)
<223>  BNA


<220>
<221>  misc_feature
<222>  (14)..(15)
<223>  BNA, n=5-mC


<400>  40
ttgtcctgct aatntt                                                    16


<210>  41
<211>  16
<212>  DNA
<213>  Artificial

<220>
<223>  Antisense oligonucleotide: ARHGAP-1931-BNA-16-cont3

<220>
<221>  misc_feature
<222>  (1)..(3)
<223>  BNA, n=5-mC


<220>
<221>  misc_feature
<222>  (13)..(15)
<223>  BNA, n=5-mC


<400>  41
tntcaatgcc tgtttt                                                    16

SEQUENCE LISTING

<110>  Osaka University

<120>  NOVEL ANTITUMOR AGENT AND METHOD FOR SCREENING SAME

<130>  OSUN-001-EP

<140>  13740969.4
<141>  2013-01-28

<150>  JP 2012-15982
<151>  2012-01-27

<160>  41

<170>  PatentIn version 3.5

<210>  1
<211>  58
<212>  DNA
<213>  Artificial

<220>
<223>  shRNA ARHGAP #1

<400>  1
ccggcggtat cagttcacat cgatactcga gtatcgatgt gaactgatac cgtttttg          58


<210>  2
<211>  58
<212>  DNA
<213>  Artificial

<220>
<223>  shRNA ARHGAP #2

<400>  2
ccggccttct attacacctc aagaactcga gttcttgagg tgtaatagaa ggtttttg          58


<210>  3
<211>  57
<212>  DNA
<213>  Artificial

<220>
<223>  shRNA Control

<400>  3
ccggcaacaa gatgaagagc accaactcga gttggtgctc ttcatcttgt tgttttt           57


<210>  4
<211>  16
<212>  DNA
<213>  Artificial

<220>
<223>  Antisense oligonucleotide: ARHGAP-47-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  4
   tnngccccca gctnnt                                                    16


<210>  5
<211>  16
<212>  DNA
<213>  Artificial

<220>
<223>  Antisense oligonucleotide: ARHGAP-126-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  5
ntnccccatc agnntg                                                     16


<210>  6
<211>  16
<212>  DNA
<213>  Artificial

<220>
<223>  Antisense oligonucleotide: ARHGAP-203-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  6
ntnaggcaac tcttnc                                                     16


<210>  7
<211>  16
<212>  DNA

<213>  Artificial

<220>
<223>  Antisense oligonucleotide: ARHGAP-382-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  7
tntattgcca gattnt                                                       16


<210>  8
<211>  16
<212>  DNA
<213>  Artificial

<220>
<223>  Antisense oligonucleotide: ARHGAP-406-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  8
ntttctgatt ctttng                                                       16


<210>  9
<211>  16
<212>  DNA
<213>  Artificial

<220>
<223>  Antisense oligonucleotide: ARHGAP-591-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  9

nnngtctggc acttnt                                                        16


<210>  10
<211>  16
<212>  DNA
<213>  Artificial

<220>
<223>  Antisense oligonucleotide: ARHGAP-625-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  10
ntnaaatttg aantnc                                                        16


<210>  11
<211>  16
<212>  DNA
<213>  Artificial

<220>
<223>  Antisense oligonucleotide: ARHGAP-720-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  11
tntgatccca cattnc                                                        16


<210>  12
<211>  16
<212>  DNA
<213>  Artificial

<220>
<223>  Antisense oligonucleotide: ARHGAP-843-BNA-16


<220>
<221>  misc_feature
<222>  (1)..(3)
<223>  BNA

<220>
<221>  misc_feature
<222>  (13)..(15)
<223>  BNA

<400>  12
ttttaccccc tatttc                                                        16


<210>  13
<211>  16
<212>  DNA
<213>  Artificial

<220>
<223>  Antisense oligonucleotide: ARHGAP-969-BNA-16


<220>
<221>  misc_feature
<222>  (1)..(3)
<223>  BNA, n=5-mC

<220>
<221>  misc_feature
<222>  (13)..(15)
<223>  BNA

<400>  13
tnngaaaaag ccnttc                                                        16


<210>  14
<211>  16
<212>  DNA
<213>  Artificial

<220>
<223>  Antisense oligonucleotide: ARHGAP-1006-BNA-16


<220>
<221>  misc_feature
<222>  (1)..(3)
<223>  BNA, n=5-mC

<220>
<221>  misc_feature
<222>  (13)..(15)
<223>  BNA

<400>  14
ttntttagtg ctttta                                                        16


<210>  15
<211>  16
<212>  DNA
<213>  Artificial

<220>
<223>  Antisense oligonucleotide: ARHGAP-1162-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  15
ttttcctctg tgnnta                                                       16


<210>  16
<211>  15
<212>  DNA
<213>  Artificial

<220>
<223>  Antisense oligonucleotide: ARHGAP-1344-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  16
tnttttcatg tcntt                                                        15


<210>  17
<211>  16
<212>  DNA
<213>  Artificial

<220>
<223>  Antisense oligonucleotide: ARHGAP-1447-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  17
tnnaggataa aatntg                                                       16


<210>  18
<211>  16

<212>  DNA
<213>  Artificial

<220>
<223>  Antisense oligonucleotide: ARHGAP-1538-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  18
ttnaccagga gtttna                                                       16


<210>  19
<211>  16
<212>  DNA
<213>  Artificial

<220>
<223>  Antisense oligonucleotide: ARHGAP-1748-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  19
nttgatggac ttnntt                                                       16


<210>  20
<211>  16
<212>  DNA
<213>  Artificial

<220>
<223>  Antisense oligonucleotide: ARHGAP-1849-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  20
ntntgagatg acnntt                                                        16


<210>  21
<211>  16
<212>  DNA
<213>  Artificial

<220>
<223>  Antisense oligonucleotide: ARHGAP-1931-BNA-16


<220>
<221>  misc_feature
<222>  (1)..(3)
<223>  BNA

<220>
<221>  misc_feature
<222>  (13)..(15)
<223>  BNA, n=5-mC

<400>  21
tttgcctgca attntt                                                        16


<210>  22
<211>  16
<212>  DNA
<213>  Artificial

<220>
<223>  Antisense oligonucleotide: ARHGAP-2032-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  22
nntagattga atttna                                                        16


<210>  23
<211>  16
<212>  DNA
<213>  Artificial

<220>
<223>  Antisense oligonucleotide: ARHGAP-2176-BNA-16


<220>
<221>  misc_feature
<222>  (1)..(3)
<223>  BNA

<220>
<221>  misc_feature
<222>  (13)..(15)
<223>  BNA, n=5-mC

<400>  23
ttttcatcaa catntg                                                        16


<210>  24
<211>  16
<212>  DNA
<213>  Artificial

<220>
<223>  Antisense oligonucleotide: ARHGAPv1-2262-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  24
tnnggtaatt tgttnc                                                        16


<210>  25
<211>  16
<212>  DNA
<213>  Artificial

<220>
<223>  Antisense oligonucleotide: ARHGAPv1-2311-BNA-16


<220>
<221>  misc_feature
<222>  (1)..(3)
<223>  BNA

<220>
<221>  misc_feature
<222>  (13)..(15)
<223>  BNA, n=5-mC

<400>  25
tttgcatcta cttntt                                                        16


<210>  26
<211>  16
<212>  DNA
<213>  Artificial

<220>
<223>  Antisense oligonucleotide: ARHGAPv1-2628-BNA-16

<220>
<221>  misc_feature
<222>  (1)..(3)
<223>  BNA, n=5-mC

<220>
<221>  misc_feature
<222>  (13)..(15)
<223>  BNA, n=5-mC

<400>  26
nntctgggct atnttc                                                    16


<210>  27
<211>  16
<212>  DNA
<213>  Artificial

<220>
<223>  Antisense oligonucleotide: ARHGAPv1-2808-BNA-16


<220>
<221>  misc_feature
<222>  (1)..(3)
<223>  BNA

<220>
<221>  misc_feature
<222>  (13)..(15)
<223>  BNA, n=5-mC

<400>  27
tttcatgttc catntt                                                    16


<210>  28
<211>  16
<212>  DNA
<213>  Artificial

<220>
<223>  Antisense oligonucleotide: ARHGAPv1-2942-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  28
ttncatcata ttntna                                                    16


<210>  29

<211>  16
<212>  DNA
<213>  Artificial

<220>
<223>  Antisense oligonucleotide: ARHGAPv1-3278-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  29
nnntgtaggt tgtntg                                                        16


<210>  30
<211>  16
<212>  DNA
<213>  Artificial

<220>
<223>  Antisense oligonucleotide: ARHGAPv1-3484-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  30
ttngagggta actnna                                                        16


<210>  31
<211>  16
<212>  DNA
<213>  Artificial

<220>
<223>  Antisense oligonucleotide: ARHGAPv1-4399-BNA-16


<220>
<221>  misc_feature
<222>  (1)..(3)
<223>  BNA

<220>
<221>  misc_feature
<222>  (13)..(15)
<223>  BNA, n=5-mC

<400>  31
nttgctccat tctttc                                                       16


<210>  32
<211>  16
<212>  DNA
<213>  Artificial

<220>
<223>  Antisense oligonucleotide: ARHGAPv1-5140-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  32
nttcctctac aannta                                                       16


<210>  33
<211>  15
<212>  DNA
<213>  Artificial

<220>
<223>  Antisense oligonucleotide: ARHGAPv1-5194-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  33
tntaacaacc aatnt                                                        15


<210>  34
<211>  16
<212>  DNA
<213>  Artificial

<220>
<223>  Antisense oligonucleotide: ARHGAPv2-2215-BNA-16


<220>
<221>  misc_feature
<222>  (1)..(3)

<223>  BNA, n=5-mC

<220>
<221>  misc_feature
<222>  (13)..(15)
<223>  BNA

<400>  34
ntntaacagt agtatg                                                       16


<210>  35
<211>  16
<212>  DNA
<213>  Artificial

<220>
<223>  Antisense oligonucleotide: ARHGAPv2-2285-BNA-16


<220>
<221>  misc_feature
<222>  (1)..(3)
<223>  BNA, n=5-mC

<220>
<221>  misc_feature
<222>  (13)..(15)
<223>  BNA

<400>  35
tntagaacag taaatt                                                       16


<210>  36
<211>  16
<212>  DNA
<213>  Artificial

<220>
<223>  Antisense oligonucleotide: ARHGAPv2-2306-BNA-16


<220>
<221>  misc_feature
<222>  (1)..(3)
<223>  BNA, n=5-mC

<220>
<221>  misc_feature
<222>  (13)..(15)
<223>  BNA, n=5-mC

<400>  36
ttnaaacatg aanttt                                                       16


<210>  37
<211>  16
<212>  DNA
<213>  Artificial

<220>

<223>  Antisense oligonucleotide: ARHGAPv2-2355-BNA-16

<220>
<221>  misc_feature
<222>  (1)..(3)
<223>  BNA, n=5-mC

<220>
<221>  misc_feature
<222>  (13)..(15)
<223>  BNA

<400>  37
tnncaattgt tgatag                                                    16

<210>  38
<211>  16
<212>  DNA
<213>  Artificial

<220>
<223>  Antisense oligonucleotide: ARHGAPv2-2404-BNA-16

<220>
<221>  misc_feature
<222>  (1)..(3)
<223>  BNA

<220>
<221>  misc_feature
<222>  (13)..(15)
<223>  BNA

<400>  38
ttttaacata agaatg                                                    16

<210>  39
<211>  16
<212>  DNA
<213>  Artificial

<220>
<223>  Antisense oligonucleotide: ARHGAP-1931-BNA-16-cont1

<220>
<221>  misc_feature
<222>  (1)..(1)
<223>  BNA

<220>
<221>  misc_feature
<222>  (3)..(3)
<223>  BNA

<220>
<221>  misc_feature
<222>  (7)..(7)
<223>  BNA

<220>
<221>  misc_feature
<222>  (12)..(12)
<223>  BNA, n=5-mC

<220>
<221>  misc_feature
<222>  (14)..(14)
<223>  BNA, n=5-mC

<220>
<221>  misc_feature
<222>  (15)..(15)
<223>  BNA

<400>  39
tttgcctgca attntt                                                    16


<210>  40
<211>  16
<212>  DNA
<213>  Artificial

<220>
<223>  Antisense oligonucleotide: ARHGAP-1931-BNA-16-cont2


<220>
<221>  misc_feature
<222>  (1)..(1)
<223>  BNA

<220>
<221>  misc_feature
<222>  (4)..(4)
<223>  BNA

<220>
<221>  misc_feature
<222>  (7)..(7)
<223>  BNA

<220>
<221>  misc_feature
<222>  (10)..(10)
<223>  BNA

<220>
<221>  misc_feature
<222>  (14)..(15)
<223>  BNA, n=5-mC

<400>  40
ttgtcctgct aatntt                                                    16


<210>  41
<211>  16
<212>  DNA
<213>  Artificial

<220>
<223>  Antisense oligonucleotide: ARHGAP-1931-BNA-16-cont3

<220>
<221>  misc_feature
<222>  (1)..(3)
<223>  BNA, n=5-mC

<220>
<221>  misc_feature
<222>  (13)..(15)
<223>  BNA, n=5-mC

<400>  41
tntcaatgcc tgtttt                                                    16